# EUROPEAN PATENT SPECIFICATION

(11) **EP 3 253 335 B1**
(45) Date of publication and mention of the grant of the patent: **14.08.2019**
(21) Application number: 16747395.8
(22) Date of filing: 05.02.2016
(51) Int. Cl.: A61F 2/44, A61F 2/30, A61F 2/46

(54) **EXPANDABLE, ADJUSTABLE INTER-BODY FUSION DEVICES**
EXPANDIERBARE, ANPASSBARE INTERSOMATISCHE FUSIONSVORRICHTUNGEN
DISPOSITIFS D'ARTHRODÈSE AJUSTABLES ET EXTENSIBLES

(30) Priority: 05.02.2015 US 201562112332 P
(43) Date of publication of application: 13.12.2017
(73) Proprietor: Spectrum Spine IP Holdings, LLC, Atlanta, GA 30339 (US)
(72) Inventor: ROBINSON, James, C., Atlanta, GA 30339 (US)
(74) Representative: Van Breda, Jacobus
(86) International application number: PCT/US2016/016899
(87) International publication number: WO 2016/127139

(56) References cited:
- WO-A1-2013/158294
- US-A1- 2010 292 796
- US-A1- 2011 282 453
- US-A1- 2011 282 453
- US-A1- 2014 121 774
- US-B1- 8 940 049

## Description

### Field of the Invention

This invention relates generally to spinal surgery, and more particularly to devices and methods for stabilization of the spine in association with placement of an expandable inter-body construct with an adjustable construct angle for inter-body fusion or the like.

### Background of the Invention

Damage or disease that affects the spinal disc within an individual's spinal column may lead to neurologic impairment with possible permanent damage to the surrounding tissue. Maintaining proper anatomic spacing and lordosis within the spine is critical to ensuring continued functionality of the surrounding tissue and for the spinal column, the spinal cord and nerve roots and therefore, avoidance of long term serious neurological impairment.

Typically, spinal implants that are used as a spacer type of device have a fixed overall length and are implanted without the ability to adjust the degree of expansion or curvature without using multiple insertion instrumentation. Some of the known procedures for introducing spinal implants comprise Anterior Lumbar Inter-body Fusion (" ALIF"), Lateral Lumbar Inter-body Fusion ("LLIF"), Posterior Lumbar Interbody Fusion ("PLIF"), Oblique Lumbar Inter-body Fusion ("OLIF"), Direct Lateral Fusion ("DLIF"), Transforaminal Lumbar Inter-body Fusion ("TLIF"), and the like. A need remains for an expandable, adjustable spacer type of implant that allows the surgeon to insert the implant in an unexpanded position to minimize the size of the surgical incision, facilitate the operative technique and decrease patient morbidity.

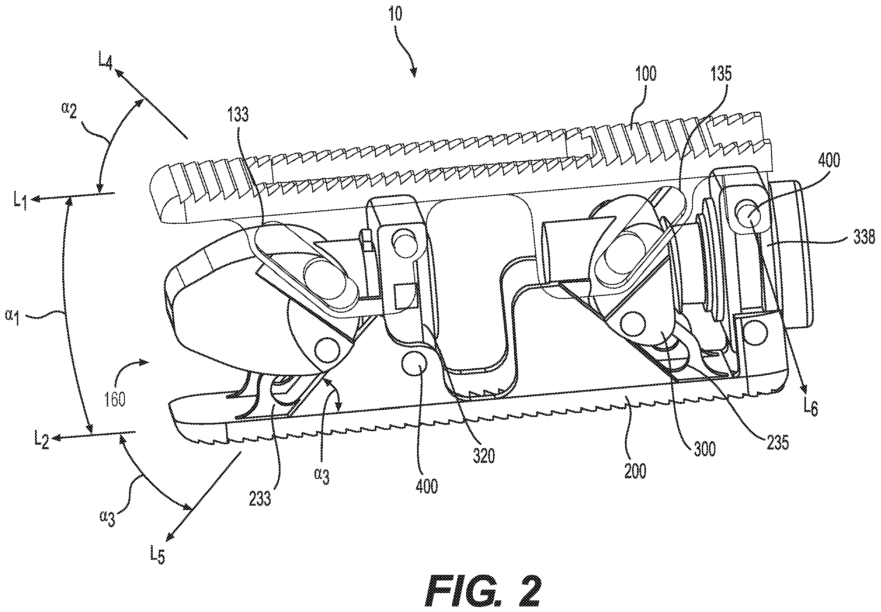

US2014/121774 discloses an expandable fusion device capable of being installed inside an intervertebral disc space to maintain normal disc spacing and restore spinal stability, thereby facilitating an intervertebral fusion. In one embodiment, the fusion device includes a body portion, a first endplate, and a second endplate. The first and second endplates are capable of being moved in a direction away from the body portion into an expanded configuration or capable of being moved towards the body portion into an unexpanded configuration. The expandable fusion device is capable of being deployed and installed in the unexpanded configuration or the expanded configuration. With reference to FIG. 2, an exploded perspective view of one embodiment of the fusion device is shown. In an exemplary embodiment, the fusion device includes a body portion, a first endplate, a second endplate, a translation member, a plurality of pins, an actuation member, and a locking mechanism. The translation member is sized to be received within a central opening of the body portion and includes at least a first expansion portion.

US2011/282453 discloses an implantable orthopedic stability device that can be inserted into a target site with the device in a compressed or contracted (i.e., small) configuration. Once positioned in the deployment site, the device can be transformed into an expanded (i.e., larger, bigger) configuration. The device can be inserted and expanded in orthopedic target sites for fixation and/or support.

### SUMMARY

Presented herein is an inter-body fusion device, or implant, for use in spinal surgery. In one aspect, the inter-body fusion device can be an expandable fusion device having an expandable height and volume. In another aspect, the inter-body fusion
device can be an adjustable fusion device such that an angle formed between an upper bone contact surface and a lower bone contact surface is selectively adjustable by the surgeon.

In one aspect, the inter-body fusion device comprises a first plate, a second plate, and an insert positioned substantially therebetween the first plate and the second plate. The first plate, the second plate, and the insert define an interior cavity. In one aspect, moving at least a portion of the insert longitudinally with respect to the first and second plates in a first direction increases the distance between the first plate relative to the second plate, effectively expanding the inter-body fusion device and increasing the volume of the interior cavity. In another aspect, moving at least a portion of the insert longitudinally with respect to the first and second plates in the first direction increases the angle formed between the first plate relative to the second plate.

It is contemplated that this technology can be used for a variety of implants used for a variety of spinal procedures. These procedures include, but are not limited to OLIF (anterior or posterior), DLIF, PLIF, TLIF, ALIF, and LLIF. So, depending upon the procedure and point of insertion for the implant, the geometry of the implant can differ.

In an exemplified aspect, at least one of the first plate and the second plate define at least one graft window that is in communication with the interior cavity.

Also presented herein is a (not claimed) method of using an inter-body fusion device during an inter-body fusion procedure. In one aspect, the method comprises accessing the desired disc space, choosing the correct insert size with the appropriate height range, inserting the inter-body fusion device into the desired area in the disc space, expanding the inter-body fusion device from a first unexpanded position to a second expanded position and adjusting the angle formed between the first plate relative to the second plate to a desired angle. An additional step of packing the interior cavity via with bone fusion material either prior to or after expansion is also contemplated.

Related methods of operation are also provided. Other apparatuses, methods, systems, features, and advantages of the inter-body fusion device and the method of its use will be or become apparent to one with skill in the art upon examination of the following figures and detailed description. It is intended that all such apparatuses, systems, features, and advantages included within this description, be within the scope of the inter-body fusion device and be protected by the accompanying claims.

### DESCRIPTION OF THE FIGURES

The accompanying drawings, which are incorporated in and constitute a part of this specification, illustrate certain aspects of the instant invention and together with the description, serve to explain, without limitation, the principles of the invention. Like reference characters used therein indicate like parts throughout the several drawings.
FIG. 1 is a front perspective view of one embodiment of an expandable, adjustable inter-body fusion device in a second expanded position, the device comprising a first plate, a second plate and an insert, according to one aspect;
FIG. 2 is a front perspective of the inter-body fusion device of FIG. 1 in the second expanded position, in which a device angle between the first plate and the second plate is substantially 0 degrees (the first plate and the second plate are substantially parallel), and in which the first plate is illustrated transparently for clarity;
FIG. 3 is a front perspective of the inter-body fusion device of FIG. 1 in a first unexpanded position;
FIG. 4 is a front perspective of the inter-body fusion device of FIG. 1 in the second expanded position, and in which the device angle between the first plate and the second plate is greater than 0 degrees;
FIG. 5 is front perspective view of the insert of FIG. 1, according to one aspect;
FIG. 6 is a side elevational view of the insert of FIG. 5;
FIG. 7 is a rear perspective view of the insert of FIG. 5;
FIG. 7A is a rear perspective view of a first member of the insert of FIG. 5.
FIG. 7B is a rear perspective view of a second member of the insert of FIG. 5.
FIG. 8 is a front perspective of the inter-body fusion device of FIG. 1 in the second expanded position, in which the device angle between the first plate and the second plate is substantially 0 degrees, and in which the first plate is illustrated transparently for clarity, showing the inter-body fusion device coupled to a device driver, according to one aspect;
FIG. 9 is a front perspective of the inter-body fusion device of FIG. 1 in the second expanded position, in which the device angle between the first plate and the second plate is greater than 0 degrees, and in which the first plate is illustrated transparently for clarity, showing the inter-body fusion device coupled to a device driver, according to one aspect;
FIG. 10 is a front perspective of the inter-body fusion device of FIG. 1 in the first unexpanded position, in which the first plate is illustrated transparently for clarity, showing the inter-body fusion device coupled to a device driver, according to one aspect;
FIG. 11 is a rear perspective of the inter-body fusion device of FIG. 1 in the second expanded position, in which the first plate is illustrated transparently for clarity, showing the inter-body fusion device coupled to a device driver, according to one aspect;
FIG. 12 is side elevational of the device driver of FIG. 9, showing the device driver coupled to the inter-body fusion device of FIG. 1, according to one aspect;
FIG. 13 is a front perspective view of a second embodiment of an expandable, adjustable inter-body fusion device in a first unexpanded position, the device comprising a first plate, a second plate and an insert, according to one aspect;
FIG. 14 is a perspective view of the inter-body fusion device of FIG. 13 in the first unexpanded position, in which the first plate is illustrated transparently for clarity;
FIG. 15 is a perspective view of the inter-body fusion device of FIG. 13 in a second expanded position in which the device angle between the first plate and the second plate is substantially 0 degrees.
FIG. 16 is a perspective view of the inter-body fusion device of FIG. 13 in the second expanded position in which the device angle between the first plate and the second plate is substantially 0 degrees and in which the first plate is illustrated transparently for clarity;
FIG. 17 is a perspective view of the inter-body fusion device of FIG. 13 in the second expanded position and in which the device angle between the first plate and the second plate is greater than 0 degrees;
FIG. 18 is a perspective view of the inter-body fusion device of FIG. 13 in the second expanded position, in which the device angle between the first plate and the second plate is greater than 0 degrees and in which the first plate is illustrated transparently for clarity;
FIG. 19 is a perspective view of the insert of the inter-body fusion device of FIG. 13, according to one aspect;
FIG. 20 is a side elevational view of the insert of FIG. 19;
FIG. 21 is a front perspective view of a third embodiment of an expandable, adjustable inter-body fusion device in a second expanded position, the device comprising a first plate, a second plate and an insert, and in which a device angle between the first plate and the second plate is substantially 0 degrees, according to one aspect;
FIG. 22 is a perspective view of the inter-body fusion device of FIG. 21 in a first unexpanded position, in which the first plate is illustrated transparently for clarity;
FIG. 23 is a perspective view of the inter-body fusion device of FIG. 21 in the second expanded position, in which the device angle between the first plate and the second plate is greater than 0 degrees and in which the first plate is illustrated transparently for clarity;
FIG. 24 is a front perspective view of a fourth embodiment of an expandable, adjustable inter-body fusion device in a second expanded position, the device comprising a first plate, a second plate and an insert, and in which a device angle between the first plate and the second plate is substantially 0 degrees, according to one aspect;
FIG. 25 is a perspective view of the inter-body fusion device of FIG. 24 in a first unexpanded position, in which the first plate is illustrated transparently for clarity;
FIG. 26 is a perspective view of the inter-body fusion device of FIG. 24 in the second expanded position, in which the device angle between the first plate and the second plate is greater than 0 degrees and in which the first plate is illustrated transparently for clarity;
FIG. 27 is a front perspective view of the inter-body fusion device of FIG. 24 in the second expanded position, in which a device angle between the first plate and the second plate is substantially 0 degrees, and in which a first member of the insert is separate from the second member of the insert, according to one aspect;
FIG. 28 is a perspective view of the inter-body fusion device of FIG. 27 in the second expanded position, in which the first plate is illustrated transparently for clarity; and
FIG. 29 is a perspective view of the inter-body fusion device of FIG. 27 in the second expanded position, in which the device angle between the first plate and the second plate is greater than 0 degrees and in which the first plate is illustrated transparently for clarity.

### DESCRIPTION OF THE INVENTION

The present invention can be understood more readily by reference to the following detailed description, examples, and claims, and their previous and following description. Before the present system, devices, and/or methods are disclosed and described, it is to be understood that this invention is not limited to the specific systems, devices, and/or methods disclosed unless otherwise specified, as such can, of course, vary. It is also to be understood that the terminology used herein is for the purpose of describing particular aspects only and is not intended to be limiting.

The following description of the invention is provided as an enabling teaching of the invention in its best, currently known aspect. Those skilled in the relevant art will recognize that many changes can be made to the aspects described, while still obtaining the beneficial results of the present invention. It will also be apparent that some of the desired benefits of the present invention can be obtained by selecting some of the features of the present invention without utilizing other features. Accordingly, those who work in the art will recognize that many modifications and adaptations to the present invention are possible and can even be desirable in certain circumstances and are a part of the present invention. Thus, the following description is provided as illustrative of the principles of the present invention and not in limitation thereof.

As used herein, the singular forms "a," "an" and "the" include plural referents unless the context clearly dictates otherwise. Thus, for example, reference to a "plate" includes aspects having two or more plates unless the context clearly indicates otherwise.

Ranges can be expressed herein as from "about" one particular value, and/or to "about" another particular value. When such a range is expressed, another aspect includes from the one particular value and/or to the other particular value. Similarly, when values are expressed as approximations, by use of the antecedent "about," it will be understood that the particular value forms another aspect. It will be further understood that the endpoints of each of the ranges are significant both in relation to the other endpoint, and independently of the other endpoint.

As used herein, the terms "optional" or "optionally" mean that the subsequently described event or circumstance may or may not occur, and that the description includes instances where said event or circumstance occurs and instances where it does not.

Terms used herein, such as "exemplary" or "exemplified," are not meant to show preference, but rather to explain that the aspect discussed thereafter is merely one example of the aspect presented.

Additionally, as used herein, relative terms, such as "substantially", "generally", "approximately", and the like, are utilized herein to represent an inherent degree of uncertainty that may be attributed to any quantitative comparison, value, measurement, or other representation. These terms are also utilized herein to represent the degree by which a quantitative representation may vary from a stated reference without resulting in a change in the basic function of the subject matter at issue.

In one aspect, presented herein is an inter-body fusion device for use in spinal surgery, such as, but not limited to, ALIF, OLIF, TLIF, LLIF, PLIF, and DLIF procedures. In another aspect, the inter-body fusion device can be an expandable inter-body fusion device such that a height of the device can be selectively adjusted by a user, such as a surgeon. In a further aspect, the inter-body fusion device can be an adjustable fusion device such that a device angle formed between an upper bone contact surface and a lower bone contact surface is selectively adjustable by the user. In another aspect, the inter-body fusion device can be an expandable, adjustable inter-body fusion device having a selectively expandable height and a selectively adjustable device angle.

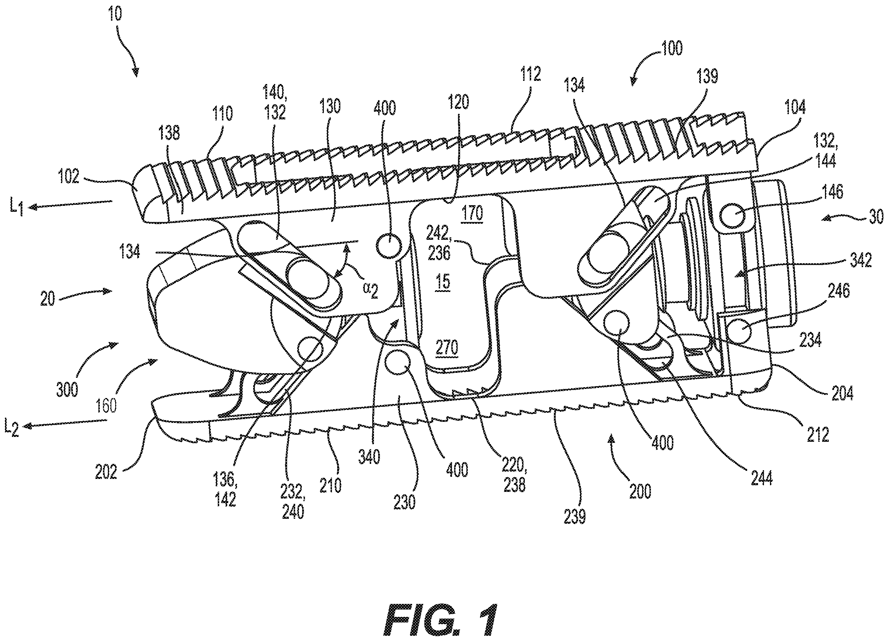

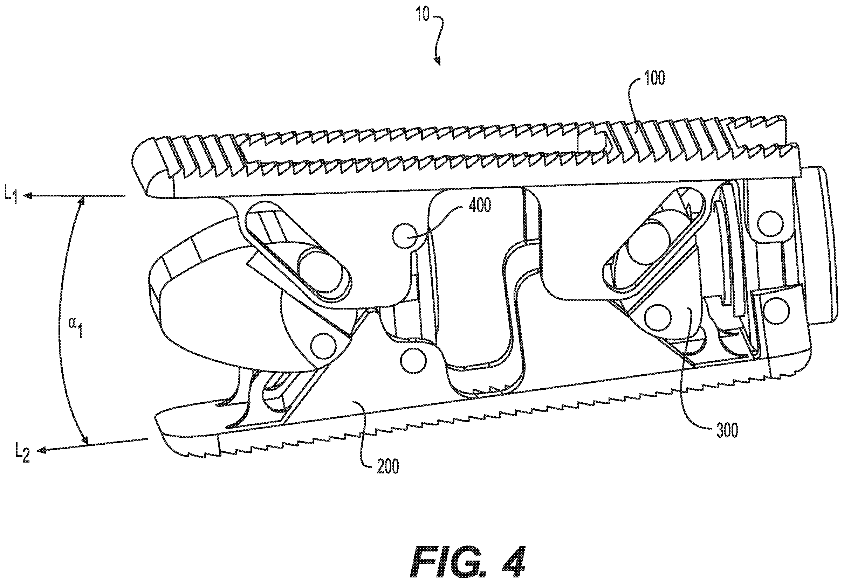

In one aspect and as illustrated in Figures 1-4, the inter-body fusion device 10 comprises a first plate 100, a second plate 200, and an insert 300 positioned substantially therebetween the first plate 100 and the second plate 200. The first plate has a leading edge 102, a trailing edge 104, an upper bone contact surface 110 and an opposed first plate inner surface 120. The second plate 200 has a leading edge 202, a trailing edge 204, a lower bone contact surface 210 and an opposed second plate inner surface 220. In one aspect, the first plate 100, the second plate 200, and the insert 300 define an interior cavity 15.

In one aspect, moving at least a portion of the insert 300 longitudinally with respect to the first plate 100 and the second plate 200 (that is, either toward the leading end 20 or toward the trailing end 30 of the device) can increase the distance between the first plate relative to the second plate, effectively expanding the inter-body fusion device and increasing the volume of the interior cavity 15. In another aspect, a device angle α₁ formed between a longitudinal axis L₁ of the first plate 100 and a longitudinal axis L₂ of the second plate 200 can be selectively adjusted by a user to vary the volume of the interior cavity and/or better position the device 10 in the disc space. For example, the device angle α₁ can be substantially 0 degrees such that the first plate and the second plate are substantially parallel to each other. In other examples, the device angle α₁ can be an acute angle of about 1 degree, 2 degrees, 3 degrees, 4 degrees, 5 degrees, 6 degrees, 7 degrees, 8 degrees, 9 degrees, 10 degrees, 11 degrees, 12 degrees, 13 degrees, 14 degrees, 15 degrees, 20 degrees, 25 degrees, 30 degrees, 35 degrees, 40 degrees, 45 degrees or greater than about 45 degrees.

At least one of the first plate 100 and the second plate 200 has at least one longitudinal sidewall 130, 230 extending substantially between the respective inner surface 120, 220 and bone contact surface 110, 210. In one aspect, the at least one longitudinal sidewall 130, 230 comprises a plurality of longitudinal sidewalls. For example, the longitudinal sidewall can comprise two longitudinal sidewalls. In another aspect, the longitudinal sidewall(s) can be positioned substantially near a peripheral edge 139, 239 of the first and/or second plate.

In one aspect, the longitudinal sidewall 130 of the first plate 100 can define at least one slot 132 having a slot axis L₄. In another aspect, the at least one slot of the first plate can comprise a plurality of slots, such as, for example and without limitation, a first inclined slot 140 and a second inclined slot 144. Each slot can have a leading end 133 and a trailing end 135, the leading end being positioned closer to the leading edge 102 of the first plate 100. In another aspect, the first slot and/or the second slot can be positioned along the slot axis L₄ that is substantially transverse to the longitudinal axis L₁ of the first plate 100. Optionally, however, the slot axis L₄ of the first slot 140 and/or the second slot 144 can be at an acute slot angle relative to the longitudinal axis L₁ of the first plate. That is, the slot axis of the first slot 140 and/or the second slot 144 can be at an acute surface angle α₂ relative to the longitudinal axis L₁ of the first plate. For example, the surface angle α₂ can be about 1 degree, 2 degrees, 3 degrees, 4 degrees, 5 degrees, 6 degrees, 7 degrees, 8 degrees, 9 degrees, 10 degrees, 11 degrees, 12 degrees, 13 degrees, 14 degrees, 15 degrees, 20 degrees, 25 degrees, 30 degrees, 35 degrees, 40 degrees, 45 degrees or greater than about 45 degrees. In another aspect, the slot axis L₄ of the first slot 140 and the slot axis of the second slot 144 can be substantially parallel to each other. Alternatively, the slot axis of the first slot can be at an angle relative to the slot axis of the second slot. For example, the slot axis L₄ of the first slot 140 can be at an acute angle relative to the slot axis of the second slot 144, or the slot axis of the first slot can be substantially transverse to the slot axis of the second slot. The first slot 140 can be sized, shaped and positioned to engage a portion of a first member 302 of the insert 300, and the second slot can be sized, shaped and positioned to engage a portion of a second member 304 of the insert. In a further aspect, the at least one slot 132 can have an inclined slot wall 134.

In another aspect, the longitudinal sidewall of the first plate 100 can have a wall width of a predetermined thickness. The longitudinal sidewall 130 of the first plate can further comprise at least one substantially flat surface 136 that is substantially parallel to the longitudinal axis L₁ of the first plate, according to another aspect. Optionally, the longitudinal sidewall 130 of the first plate 100 can comprise an upper flat surface 138, a first inclined surface 141, a lower flat surface 142 and a second inclined surface 143. In this aspect, the upper flat surface and the lower flat surface can be spaced from each other a predetermined distance that is less than the height of the insert 300. Alternatively, the upper flat surface 138 and the lower flat surface 142 can be spaced from each other a predetermined distance that is greater than or equal to the height of the insert. In one aspect, the first inclined surface and the second inclined surface can be substantially parallel to each other. Optionally, the first inclined surface 141 can be at an angle relative to the second inclined surface 143. At least one pin bore 146 can be defined in a portion of the longitudinal sidewall 130.

In one aspect, the longitudinal sidewall 230 of the second plate 200 can define at least one slot 232 having a slot axis L₅. In another aspect, the at least one slot of the second plate can comprise a plurality of slots, such as, for example and without limitation, a first inclined slot 240 and a second inclined slot 244. Each slot can have a leading end 233 and a trailing end 235, the leading end being positioned closer to the leading edge 202 of the second plate 200. In another aspect, the first slot and/or the second slot can be positioned along the slot axis L₅ that is substantially transverse to the longitudinal axis L₂ of the second plate 200. Optionally, the slot axis L₅ of the first slot 240 and/or the second slot 244 can be at an acute slot angle relative to the longitudinal axis L₂ of the second plate. That is, at the slot axis of the first slot and/or the second slot can be at an acute surface angle α₃ relative to the longitudinal axis L₂ of the second plate. For example, the surface angle α₃ can be about 1 degree, 2 degrees, 3 degrees, 4 degrees, 5 degrees, 6 degrees, 7 degrees, 8 degrees, 9 degrees, 10 degrees, 11 degrees, 12 degrees, 13 degrees, 14 degrees, 15 degrees, 20 degrees, 25 degrees, 30 degrees, 35 degrees, 40 degrees, 45 degrees or greater than about 45 degrees. In another aspect, the slot axis L₅ of the first slot 240 and the slot axis of the second slot 244 can be substantially parallel to each other. Alternatively, the slot axis of the first slot can be at an angle relative to the slot axis of the second slot. For example, the slot axis L₅ of the first slot 240 can be at an acute angle relative to the slot axis of the second slot 244, or the slot axis of the first slot can be substantially transverse to the slot axis of the second slot. The first slot 240 can be sized, shaped and positioned to engage a portion of a pin 400 coupled to the first member 302, and the second slot 244 can be sized, shaped and positioned to engage a pin coupled to the second member 304. In a further aspect, the at least one slot 232 can have an inclined slot wall 234.

In one aspect, the longitudinal sidewall of the second plate 200 can have a slot wall width of a predetermined thickness. The longitudinal sidewall 230 of the second plate can further comprise at least one substantially flat surface 236 that is substantially parallel to the longitudinal axis L₂ of the second plate, according to another aspect. Optionally, the longitudinal sidewall 230 of the second plate 200 can comprise a lower flat surface 238, a first inclined surface 241, an upper flat surface 242 and a second inclined surface 243. In this aspect, the upper flat surface and the lower flat surface can be spaced from each other a predetermined distance that is less than the height of the insert 300. Alternatively, the upper flat surface 242 and the lower flat surface 238 can be spaced from each other a predetermined distance that is greater than or equal to the height of the insert. In one aspect, the first inclined surface and the second inclined surface can be substantially parallel to each other. Optionally, the first inclined surface 241 can be at an angle relative to the second inclined surface 243. At least one pin bore 246 can be defined in a portion of the longitudinal sidewall 230.

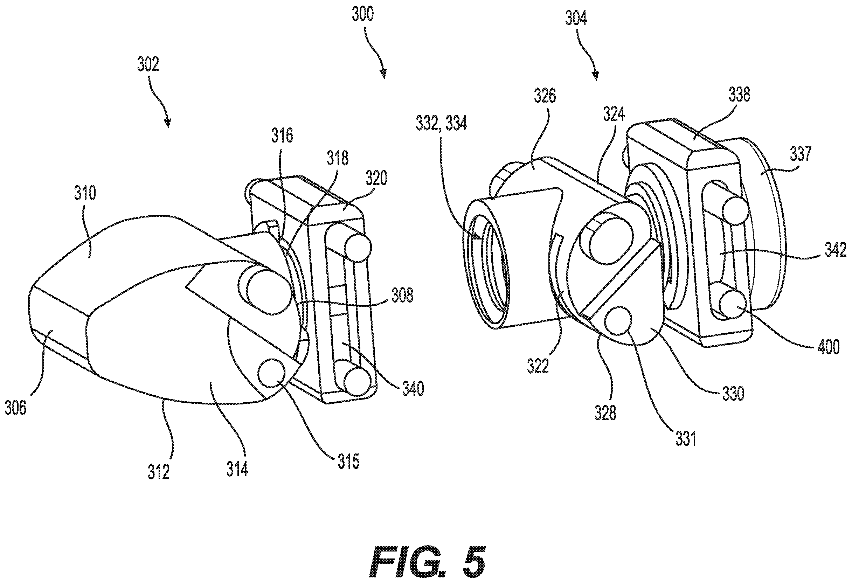

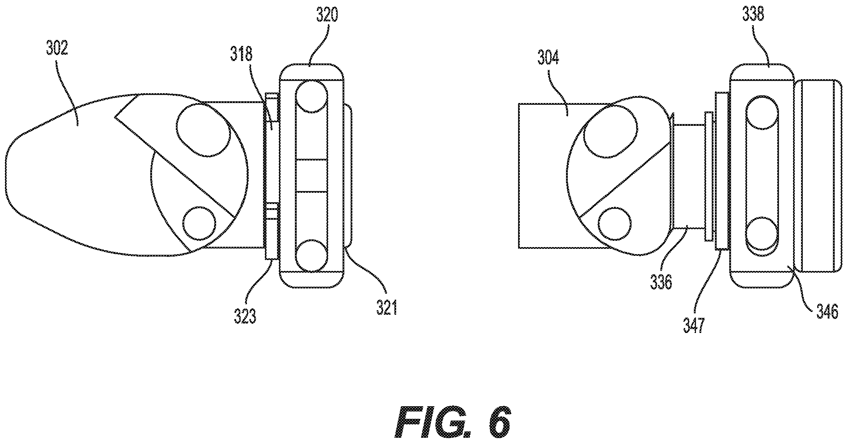

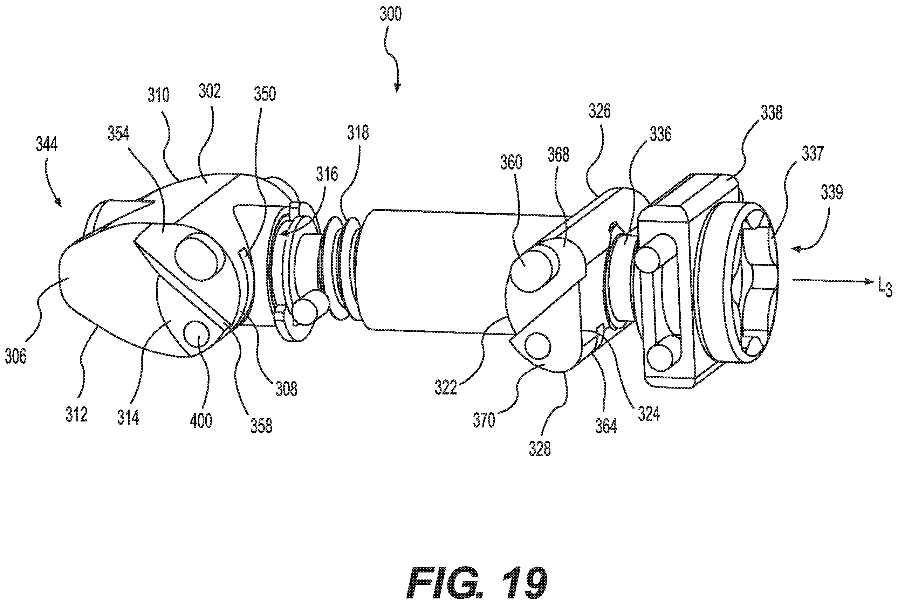

Referring now to Figures 5-7, in one exemplified aspect, the insert 300 comprises a first member 302 and a second member 304. In one aspect, the first member can be spaced from the second member a predetermined distance. In another aspect, the first member 302 can be physically separate from the second member 304, as in Figure 5. Optionally, a portion of the first member can be coupled to the second member (as illustrated in Figure 19). In one aspect, the first and second members operate independently, enabling a user to selectively alter both the distance between the first plate and the second plate and the device angle.

In one aspect, the first member 302 has a leading edge 306, a trailing edge 308, an upper plate contact surface 310 extending between the leading edge and the trailing edge, and an opposed lower plate contact surface 312 extending between the leading edge 306 and the trailing edge 308. At least one longitudinal sidewall 314 can extend substantially between the upper plate contact surface and the opposed lower plate contact surface. A first bore 316 can be defined in a portion of the trailing edge of the first member. In another aspect, at least a portion of the first bore can be threaded.

The first member 302 can comprise a first threaded shaft 318 and an optional first retainer 320, according to one aspect. The first retainer can be configured to couple a portion of the first member 302 to at least one of the first plate 100 and the second plate 200. In another aspect, the first threaded shaft can be coupled to a portion of the first retainer 320 and can be configured to complementarily engage a portion of the first bore 316. Thus, rotation of the first threaded shaft 318 can cause the distance between the trailing edge 308 of the first member 302 and the first retainer to change. For example, rotation of the first threaded shaft in a first direction can make the distance between the trailing edge of the first member and the first retainer 320 smaller. In another example, rotation of the first threaded shaft 318 in a second direction that is opposed to the first direction can make the distance between the trailing edge 308 of the first member 302 and the first retainer 320 larger. A distal end 319 of the first threaded shaft can be configured to engage an actuation device, such as a screwdriver and the like so that rotation of the actuation device can rotate the first threaded shaft 318. For example, the distal end of the first threaded shaft 318 can be slotted to engage a regular screwdriver. In another example, the distal end 319 of the first threaded shaft can be shaped to engage a hexagonal driver and the like. A shoulder 321 and/or a locking washer 323 of the second threaded shaft can engage a portion of the second retainer 338 to restrict longitudinal movement of the second threaded shaft 336.

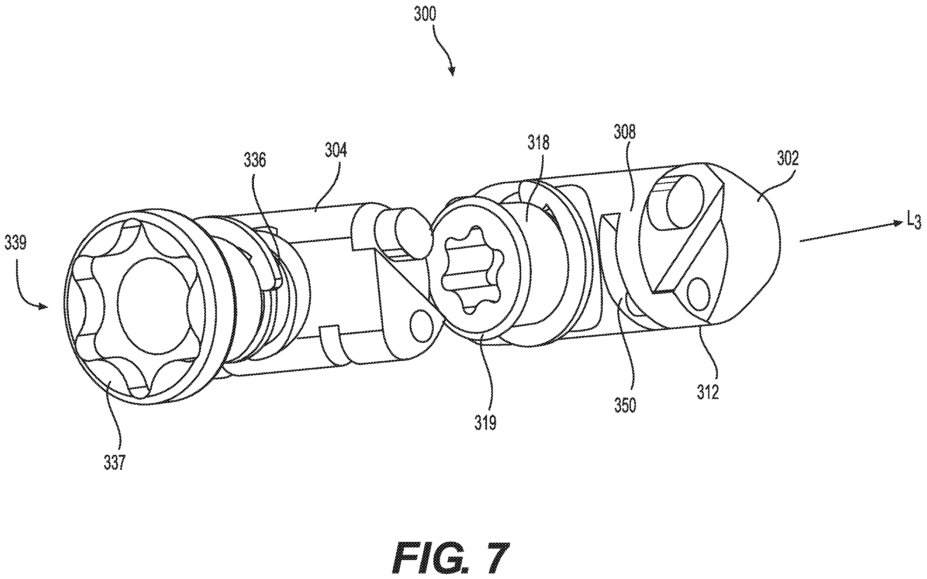

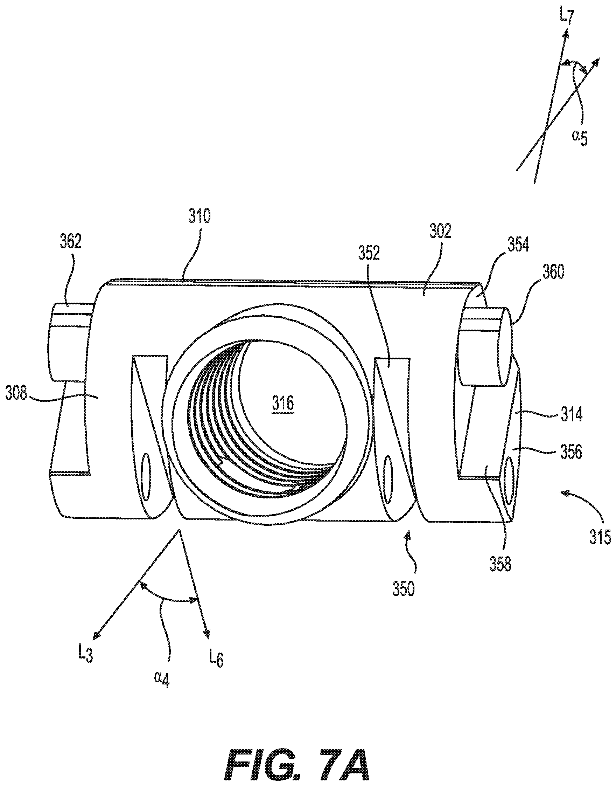

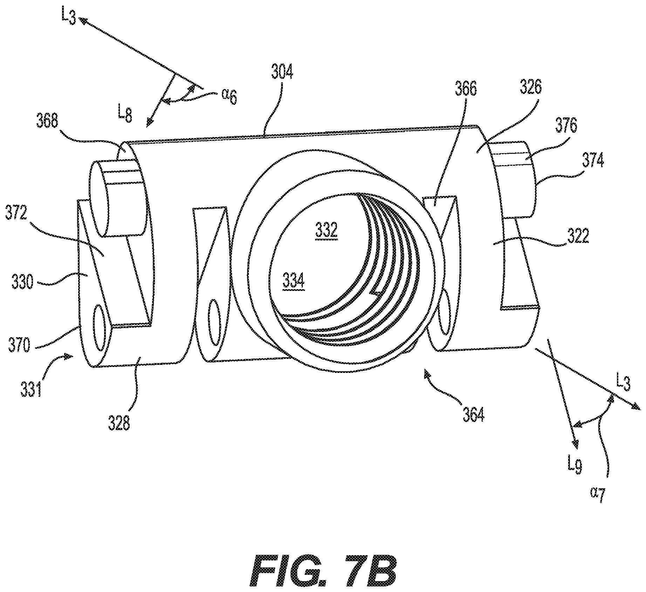

As illustrated in Figures 7 and 7A, the first member 302 can define at least one insert slot 350 in a portion of the trailing edge 308 and/or the lower plate contact surface 312. In one aspect, the at least one insert slot can have a slot axis L₆ that is in a slot plane that is substantially parallel to a longitudinal axis L₃ of the insert 300. Optionally, the at least one insert slot can comprise a plurality of insert slots such that an insert slot can be defined in the first member on each side of the first bore 316. In another aspect, each insert slot 350 can have an inclined insert slot wall 352. That is, at least a portion of the at least one insert slot of the first member 302 can be an inclined surface that is at an acute surface angle α₄ relative to the longitudinal axis L₃ of the insert. For example, the surface angle α₄ can be about 1 degree, 2 degrees, 3 degrees, 4 degrees, 5 degrees, 6 degrees, 7 degrees, 8 degrees, 9 degrees, 10 degrees, 11 degrees, 12 degrees, 13 degrees, 14 degrees, 15 degrees, 20 degrees, 25 degrees, 30 degrees, 35 degrees, 40 degrees, 45 degrees, 50 degrees, 55 degrees, 60 degrees, 65 degrees or greater than about 65 degrees. In another aspect, the insert slot 350 of the first member can have a slot width of a predetermined thickness that is greater than at least a portion of the wall thickness of the longitudinal sidewall 130, 230 of the first plate 100 and/or the second plate 200. At least one pin bore 315 can be defined in a portion of the longitudinal sidewall 314. In one aspect, the pin bore can extend through the insert slot as can be seen in Figure 7A.

In one aspect, a first portion 354 of the longitudinal sidewall 314 of the first member 302 can be recessed relative to a second portion 356. For example, the first portion of the longitudinal sidewall can be spaced from the second portion a predetermined sidewall distance. This predetermined sidewall distance can be the width of a sidewall shoulder 358 formed between the first portion 354 and the second portion 356 of the sidewall. In another aspect, at least a portion of the sidewall shoulder of the first member 302 can be an inclined surface that is at an acute surface angle α₅ relative to the longitudinal axis L₃ of the insert. For example, the surface angle α₅ can be about 1 degree, 2 degrees, 3 degrees, 4 degrees, 5 degrees, 6 degrees, 7 degrees, 8 degrees, 9 degrees, 10 degrees, 11 degrees, 12 degrees, 13 degrees, 14 degrees, 15 degrees, 20 degrees, 25 degrees, 30 degrees, 35 degrees, 40 degrees, 45 degrees, 50 degrees, 55 degrees, 60 degrees, 65 degrees or greater than about 65 degrees. In another aspect, the sidewall shoulder 358 of the first member can have a shoulder width of a predetermined thickness that is greater than, equal to, or less than at least a portion of the wall thickness of the longitudinal sidewall 130, 230 of the first plate 100 and/or the second plate 200. As can be appreciated, the sidewall shoulder can be formed on any or all longitudinal sidewalls 314 of the first member 302.

At least one tab 360 can extend from the first portion 354 of each longitudinal sidewall 314 of the first member 302, according to one aspect. In another aspect, the at least one tab can be substantially circular, substantially oval and the like. An outer wall 362 of the at least one tab can substantially align with a portion of the trailing edge 308 and/or the upper plate contact surface 310 of the first member. In a further aspect, the at least one tab 360 can have a tab length substantially equal to the width of the sidewall shoulder 358 of the first member. Optionally, the tab length can be greater than or less than the width of the sidewall shoulder of the first member 302. In still another aspect, at least a portion of the tab 360 can be spaced from and overlie the sidewall shoulder 358.

With reference again to Figures 5-7, the second member 304 has a leading edge 322, a trailing edge 324, an upper plate contact surface 326 extending between the leading edge and the trailing edge and an opposed lower plate contact surface 328 extending between the leading edge 322 and the trailing edge 324. At least one longitudinal sidewall 330 can extend substantially between the upper plate contact surface and the opposed lower plate contact surface. A second bore 332 can extend through the second member from the leading edge to the trailing edge of the second member. In another aspect, the second bore can define a longitudinal pathway 334, and at least a portion of the second bore can be threaded. For example, the second bore 332 can extend longitudinally through the second member 304, and a portion or all of the second bore can be threaded.

The second member 304 can comprise a second threaded shaft 336 and an optional second retainer 338, according to one aspect. The second retainer can be configured to couple a portion of the second member 304 to at least one of the first plate 100 and the second plate 200. In another aspect, the second threaded shaft can be coupled to a portion of the second retainer and configured to complementarily engage a portion of the second bore 332. Thus, rotation of the second threaded shaft 336 can cause the distance between the trailing edge 324 of the second member 304 and the second retainer to change. For example, rotation of the second threaded shaft in a first direction can make the distance between the trailing edge of the second member and the second retainer 338 smaller. In another example, rotation of the second threaded shaft 336 in a second direction that is opposed to the first direction can make the distance between the trailing edge 324 of the second member 304 and the second retainer 338 larger. A distal end 337 of the second threaded shaft can be configured to engage an actuation device, such as a screwdriver and the like so that rotation of the actuation device can rotate the second threaded shaft 336. For example, the distal end of the second threaded shaft can be slotted to engage a regular screwdriver. In another example, the distal end 337 can be shaped to engage a hexagonal driver and the like. In one aspect, a longitudinal duct 339 can be defined therethrough the second threaded shaft. In use, the longitudinal duct of the second threaded shaft can be substantially coaxially aligned with the longitudinal pathway 334 of the second member so that at least a portion of the actuation device can be inserted through both the longitudinal duct 339 of the second threaded shaft 336 and the longitudinal pathway 334 of the second member 304. A shoulder 346 and/or a locking washer 347 of the second threaded shaft can engage a portion of the second retainer 338 to restrict longitudinal movement of the second threaded shaft 336.

As illustrated in Figures 7 and 7B, the second member 304 can define at least one insert slot 364 in a portion of the leading edge 322 and/or the lower plate contact surface 328. In one aspect, the at least one insert slot can have a slot axis L₈ that is in a slot plane that is substantially parallel to a longitudinal axis L₃ of the insert 300. Optionally, the at least one insert slot can comprise a plurality of insert slots such that an insert slot can be defined in the second member on each side of the second bore 332. In another aspect, each insert slot 364 can have an inclined insert slot wall 366. That is, at least a portion of the at least one insert slot of the second member 304 can be an inclined surface that is at an acute surface angle α₆ relative to the longitudinal axis L₃ of the insert. For example, the surface angle α₆ can be about 1 degree, 2 degrees, 3 degrees, 4 degrees, 5 degrees, 6 degrees, 7 degrees, 8 degrees, 9 degrees, 10 degrees, 11 degrees, 12 degrees, 13 degrees, 14 degrees, 15 degrees, 20 degrees, 25 degrees, 30 degrees, 35 degrees, 40 degrees, 45 degrees, 50 degrees, 55 degrees, 60 degrees, 65 degrees or greater than about 65 degrees. In another aspect, the insert slot 364 of the second member 304 can have a slot width of a predetermined thickness that is greater than at least a portion of the wall thickness of the longitudinal sidewall 130, 230 of the first plate 100 and/or the second plate 200. At least one pin bore 331 can be defined in a portion of the longitudinal sidewall 330. In one aspect, the pin bore can extend through the insert slot 364 as can be seen in Figure 7B.

In one aspect, a first portion 368 of the longitudinal sidewall 330 of the second member 304 can be recessed relative to a second portion 370. For example, the first portion of the longitudinal sidewall can be spaced from the second portion a predetermined sidewall distance. This predetermined sidewall distance can be the width of a sidewall shoulder 372 formed between the first portion 368 and the second portion 370 of the sidewall. In another aspect, at least a portion of the sidewall shoulder of the second member 304 can be an inclined surface that is at an acute surface angle α₇ relative to the longitudinal axis L₃ of the insert 300. For example, the surface angle α₇ can be about 1 degree, 2 degrees, 3 degrees, 4 degrees, 5 degrees, 6 degrees, 7 degrees, 8 degrees, 9 degrees, 10 degrees, 11 degrees, 12 degrees, 13 degrees, 14 degrees, 15 degrees, 20 degrees, 25 degrees, 30 degrees, 35 degrees, 40 degrees, 45 degrees, 50 degrees, 55 degrees, 60 degrees, 65 degrees or greater than about 65 degrees. In another aspect, the sidewall shoulder 372 of the second member can have a shoulder width of a predetermined thickness that is greater than, equal to, or less than at least a portion of the wall thickness of the longitudinal sidewall 130, 230 of the first plate 100 and/or the second plate 200. As can be appreciated, the sidewall shoulder can be formed on any or all longitudinal sidewalls 330 of the second member.

At least one tab 374 can extend from the first portion 368 of each longitudinal sidewall 330 of the second member 304, according to one aspect. In another aspect, the at least one tab can be substantially circular, substantially oval and the like. An outer wall 376 of the at least one tab can substantially align with a portion of the leading edge 322 and/or the upper plate contact surface 326 of the second member. In a further aspect, the at least one tab 374 can have a tab length substantially equal to the width of the sidewall shoulder 372 of the second member. Optionally, the tab length can be greater than or less than the width of the sidewall shoulder of the second member 304. In still another aspect, at least a portion of the tab 374 can be spaced from and overlie the sidewall shoulder 372.

Referring again to Figures 1-4, the inter-body fusion device 10 can further comprise at least one pin 400 configured to couple a portion of the insert 300 to at least one of the first plate 100 and the second plate 200. For example, a proximal end 402 of a pin can be formed with or securedly attached to the pin bore 146 of the longitudinal sidewall 130 of the first plate and/or the pin bore 246 of the longitudinal sidewall 230 of the second plate such that a distal end 404 of the pin 400 extends from the sidewall into the interior cavity 15 of the device 10. In another example, the proximal end 402 and the distal end 404 of a pin can be formed with or securedly attached to the pin bore 315, 331 of the longitudinal sidewall 314, 330 of the first member 302 and/or the second member 304 such that a central portion 406 of the pin is positioned in the insert slot 350, 364. In one aspect, at least one pin can be positioned such that a longitudinal axis of the pin is substantially transverse to the longitudinal axis L₁ of the first plate 100. In another aspect, a portion of at least one pin 400 can be configured to slidingly engage a first slot 340 defined in the first retainer 320 and/or a second slot 342 defined in the second retainer 338 of the insert 300. In another aspect, the first slot and/or the second slot can be substantially transverse to a longitudinal axis L₃ of the insert. Optionally, however, the first slot 340 and/or the second slot 342 can be at an acute angle relative to the longitudinal axis L₃ of the insert. In still another aspect, a portion of at least one pin 400 can be configured to slidingly engage the slot wall 234 of the at least one slot 232 defined in the second plate 200.

As can be appreciated, the at least one pin 400 can comprise a plurality of pins. In one non-limiting example, the device 10 of Figures 1-4 comprises 12 pins, though it is of course contemplated that more or less pins can be used. Further, the pins can be placed in other arrangements than those shown.

In use, described more fully below, portions of the at least one tab 360, 374 of the first member 302 and/or the second member 304 can be configured to slidingly engage the inclined slot wall 134 of the slot 132 of the first plate 100 of the device 10. For example, portions of the tab 360 of the first member can be configured to be inserted into and engage a portion of the slot wall of the first slot 140 of the first plate, and portions of the tab 374 of the second member 304 can be configured to be inserted into and engage a portion of the slot wall of the second slot 144 of the first plate 100.

In another aspect, portions of the inclined insert slot wall 352, 366 of the first member 302 and/or the second member 304 can be configured to engage the first inclined surface 241 and/or the second inclined surface 243 of the second plate 200. Optionally, a pin 400 inserted through the pin bore 315, 331 of the first member and/or the second member can be configured to engage the slot wall 234 of the second plate 200. For example, a first pin 400 can be inserted through the pin bore 315 of the first member 302 and the first inclined slot 240 of the second plate so that the central portion 406 of the pin slidingly engages the inclined slot wall 234, and a second pin can be inserted through the pin bore 331 of the second member 304 and the second inclined slot 244 of the second plate 200 so that the central portion 406 of the pin slidingly engages the inclined slot wall. As seen in the Figures 1-4, the inclined slot walls of the upper and lower plates can cooperate with the pins 400 or tabs of the insert to cam or wedge the first plate 100 and/or the second plate 200 to a desired position and orientation relative to each other based on the position of the first member 302 and the second member 304 relative to the plates.

To assemble the inter-body fusion device 10, the insert 300 can be positioned between the first plate 100 and the second plate 200 such that the leading edge 306 of the insert, the leading edge 102 of the first plate, and the leading edge 202 of the second plate are facing the same direction. In one aspect, portions of the first plate 100 can overlie the second plate 200. Correspondingly, in one aspect, each longitudinal sidewall 130 of the first plate 100 can substantially align with a longitudinal sidewall 230 of the second plate 200. For example, each longitudinal sidewall of the first plate can substantially overlie at least a portion of a longitudinal sidewall of the second plate. Optionally, each longitudinal sidewall 130 of the first plate 100 can be positioned adjacent to at least a portion of a longitudinal sidewall 230 of the second plate 200 so that the inner surface 120 of the first plate and the inner surface 220 of the second plate do not contact each other. Portions of the at least one tab 360, 374 of the first member 302 and/or the second member 304 can be positioned in the inclined slot wall 134 of the slot 132 of the first plate 100 of the device 10.

A pin 400 can be inserted through at least one pin bore 315, 331 of the first member 302 and the first inclined slot 240 of the second plate so that the central portion 406 of the pin slidingly engages the inclined slot wall 234. The proximal end 402 of at least one pin 400 can be coupled to the longitudinal sidewall 130, 230 of at least one of the first and second plate 100, 200 and the distal end 404 of the pin can extend into the first slot 340 or the second slot 342 of the insert 300. Thus, when assembled, a portion of a pin can slide in the slot and allow the first plate 100, the second plate 200, and/or the insert to move relative to each other in the direction of the slot. A first pin 400 can be inserted through the pin bore 315 of the first member 302 and the first inclined slot 240 of the second plate so that the proximal and distal ends of the pin are positioned in the pin bore 315 and the central portion 406 of the pin slidingly engages the inclined slot wall 234, and a second pin can be inserted through the pin bore 331 of the second member 304 and the second inclined slot 244 of the second plate 200 so that the proximal and distal ends of the pin are positioned in the pin bore 331 and the central portion 406 of the pin is positioned in the slot 232 to slidingly engages the inclined slot wall.

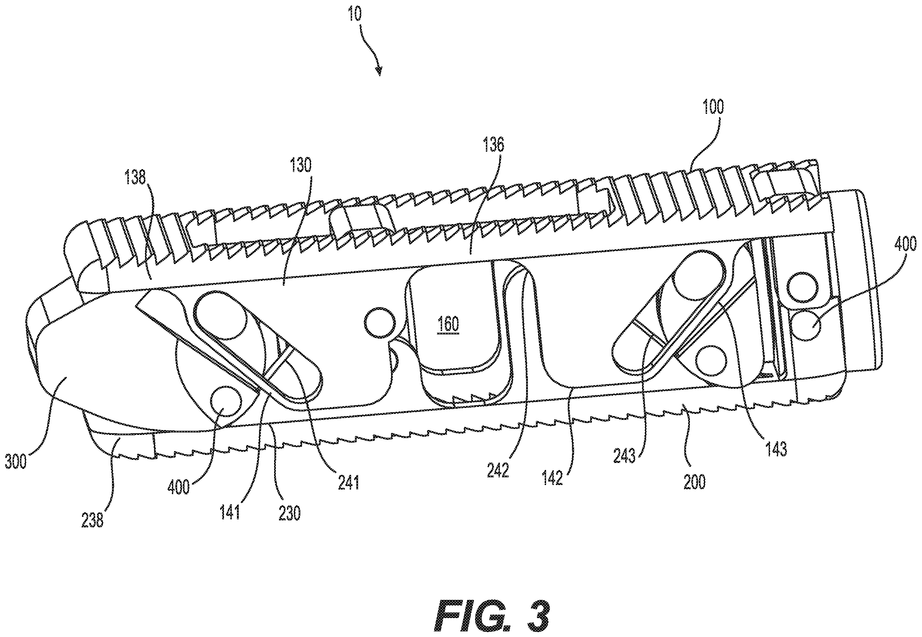

Each set of substantially aligned longitudinal sidewalls (a longitudinal sidewall 130 from the first plate 100 and a longitudinal sidewall 230 from the second plate 200) define at least one void 160, as illustrated in Figures 1 and 2. In another aspect, the at least one void can be sized and shaped to complimentarily accept a portion of the first member 302 or the second member 304 of the insert 300 therein. In this aspect, in a first unexpanded position (as illustrated in Figure 3), each of the first member and the second member of the insert 300 can be positioned substantially within the void 160 formed between the substantially aligned longitudinal sidewalls 130, 230 of the first and second plates 100, 200. In the first unexpanded position, at least one of the first member 302 and the second member 304 can be positioned in the void substantially near or in contact with the upper flat surface 138 and/or the lower flat surface 238 of the respective first and second plates. Note that the first unexpanded position is the position in which the inter-body fusion device 10 can be inserted between the adjacent vertebrae of a patient.

In one aspect, in the first unexpanded position, a portion of the longitudinal sidewall 130 of the first plate 100 and the longitudinal sidewall 220 of the second plate 200 can slide by each other until the tab 360 and/or a portion of a pin contacts the leading end 133 of the first slot 140 and/or the trailing end 135 of the second slot 144 and/or, the central portion 406 of a pin positioned in the slot 232 of the second plate contacts the leading end 233 of the first slot 240 and/or the trailing end 235 of the second slot 244. In the second expanded position, a portion of the longitudinal sidewall 130 of the first plate 100 and the longitudinal sidewall 220 of the second plate 200 can slide by each other until the tab 360 and/or a portion of a pin contacts the trailing end 135 of the first slot 140 and/or the leading end 133 of the second slot 144 and/or, the central portion 406 of a pin positioned in the slot 232 of the second plate contacts the trailing end 235 of the first slot 240 and/or the leading end 233 of the second slot 244.

The inter-body fusion device 10 can be selectively expanded about and between the first unexpanded position, in which a portion of at least one of the first member 302 and the second member 304 can be positioned in the void 160 substantially near or in contact with the upper flat surface 138 and/or the lower flat surface 238 of the respective first and second plates 100, 200, and a second expanded position in which a portion of the first member 302 and/or the second member 304 of the insert 300 can be positioned in the void a predetermined distance from the upper flat surface 138 and/or the lower flat surface 238 of the respective first and second plates 100, 200. As can be appreciated, in the second expanded position, the inter-body fusion device 10 can have a height and interior cavity 15 volume that is greater than the height and interior cavity volume of the inter-body fusion device in the first, unexpanded position. Thus, in the first unexpanded position, the interior cavity 15 of the device can have a first cavity size, and in the second expanded position the interior cavity can have a second cavity size that is greater than the first cavity size.

In one aspect, in the first, unexpanded position, the longitudinal axis L₁ of the first plate 100 and the longitudinal axis L₂ of the second plate 200 can be substantially parallel to each other or, optionally, the longitudinal axis L₁ of the first plate and the longitudinal axis L₂ of the second plate can be at an acute angle relative to each other. In another aspect, in the second, expanded position, the longitudinal axis L₁ of the first plate 100 and the longitudinal axis L₂ of the second plate 200 can be substantially parallel to each other or, optionally, the longitudinal axis L₁ of the first plate and the longitudinal axis L₂ of the second plate can be at an acute angle relative to each other.

In order to selectively expand the inter-body fusion device 10 about and between the first unexpanded position and the second expanded position, at least one of the first member 302 or the second member 304 of the insert 300 can be moved longitudinally about and between a first insert position and a second insert position. In one aspect, in the first insert position, the trailing edge 308 of the first member can be spaced from the trailing edge 104 of the first plate 100 an unexpanded first distance, and the trailing edge 324 of the second member can be spaced from the trailing edge 104 of the first plate 100 an unexpanded second distance. In the second insert position, the trailing edge 308 of the first member can be spaced from the trailing edge 104 of the first plate 100 an expanded first distance that is different than the unexpanded first distance, and the trailing edge 324 of the second member can be spaced from the trailing edge 104 of the first plate 100 an expanded second distance that is different than the unexpanded second distance. With regards to the device 10 of Figures 1-4, it can be seen that the expanded first distance is less than the unexpanded first distance, and the expanded second distance is greater than the unexpanded second distance.

In moving the inter-body fusion device 10 about and between the first unexpanded position and the second expanded position, the first member 302 and the second member 304 of the insert 300 do not necessarily need be moved simultaneously or to the same insert position. For example, the first member can be in the first insert position while the second member can be in the second insert position. In another example, the first member 302 can be in the second insert position while the second member 304 can be in the first insert position. Thus, the first member and the second member can be in any insert position between the first insert position and the second insert position at any time regardless of the position of the other member.

Upon moving the first member 302 towards the second insert position, at least a portion of the tab 360 of the first member can be moved into contact with the inclined slot wall 134 of the first inclined slot 140 of the longitudinal sidewall 130 of the first plate 100, and at least a portion of the pin 400 extending through pin bore 315 can be moved into contact with the inclined slot wall 234 of the first inclined slot 240 of the longitudinal sidewall 230 of the second plate 200. In this position, the aligned longitudinal sidewalls of the first and second plates 100, 200 can be separated by the first member traveling over the first inclined slots 140, 240 to cam the plates away from each other.

Similarly, upon moving the second member 304 towards the second insert position, at least a portion of the tab 374 of the second member can be moved into contact with the inclined slot wall 134 of the second inclined slot 144 of the longitudinal sidewall 130 of the first plate 100, and at least a portion of the pin 400 extending through the pin bore 331 can be moved into contact with the inclined slot wall 234 of the second inclined slot 244 of the longitudinal sidewall 230 of the second plate 200. In this position, the aligned longitudinal sidewalls of the first and second plates 100, 200 can be separated by the second member traveling over the second inclined slots 144, 244 to cam the plates away from each other.

As one skilled in the art can appreciate, the amount of separation achievable between the first plate 100 and the second plate 200 can be determined by at least the height of the inclined surfaces, the length of the inclined slots and the distance of longitudinal movement of the second member. Further, the angle formed between the first plate and the second plate can be determined by at least the position of the first member 302 of the insert relative to the second member 304.

It is contemplated that this technology can be used for a variety of implants used for a variety of spinal procedures. As mentioned before, these procedures include, but are not limited to OLIF, DLIF, PLIF, ALIF, TLIF, and LLIF. Because of this, depending upon the procedure and point of insertion for the implant, the geometry of the implant can differ. For example, in a DLIF expandable device, the approach is lateral. As such, the upper bone contact surface 110 can be transversely angled with respect to the lower bone contact surface 210 from a first sidewall to a second sidewall to match, increase, or decrease lordosis.

In an OLIF procedure, the inter-body fusion device 10 can be inserted obliquely, either anteriorly or posteriorly. As such, similar to the DLIF implant, the upper bone contact surface 110 can be angled transversely with respect to the lower bone contact surface 210 from the first sidewall to the second sidewall depending on the need to match, increase, or decrease lordosis. In addition, the upper bone contact surface can also be angled longitudinally with respect to the lower bone contact surface from the leading end 20 of the device to the trailing end 30.

In an exemplified aspect, at least one of the first plate 100 and the second plate 200 can define at least one graft window 170, 270 that is in communication with the interior cavity 15. The at least one graft window 170 defined in the first plate can overlie at least a portion of the at least one graft window 270 of the second plate, thereby permitting bone growth therethrough. In another aspect, the upper bone contact surface 110 of the first plate 100 comprises ridges 112 for frictionally engaging a first vertebra of the patient. As can be appreciated, the lower bone contact surface 210 of the second plate can comprise ridges 212 to frictionally engage a second vertebra of the patient.

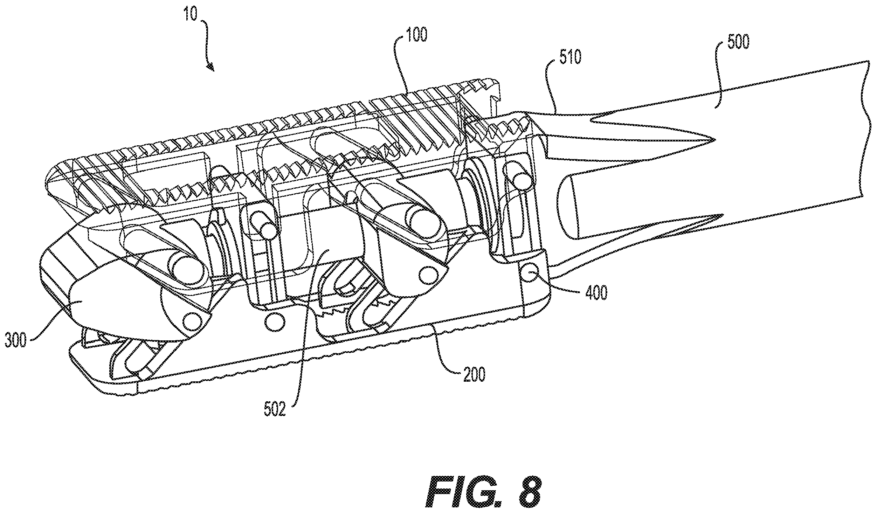

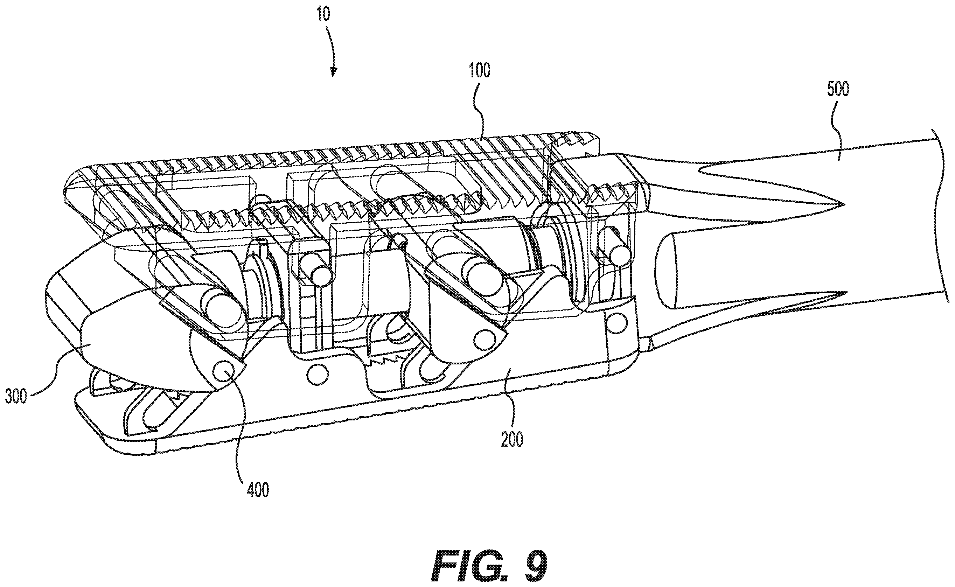

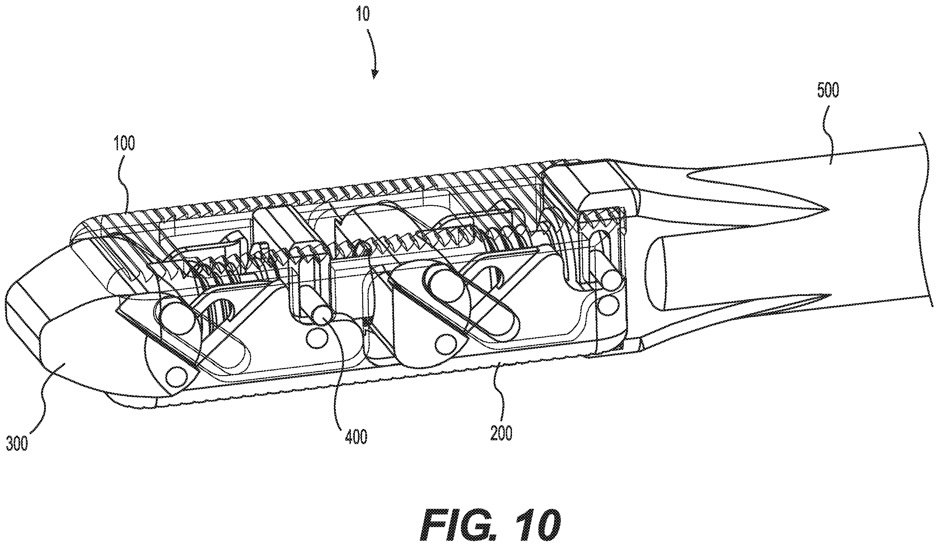

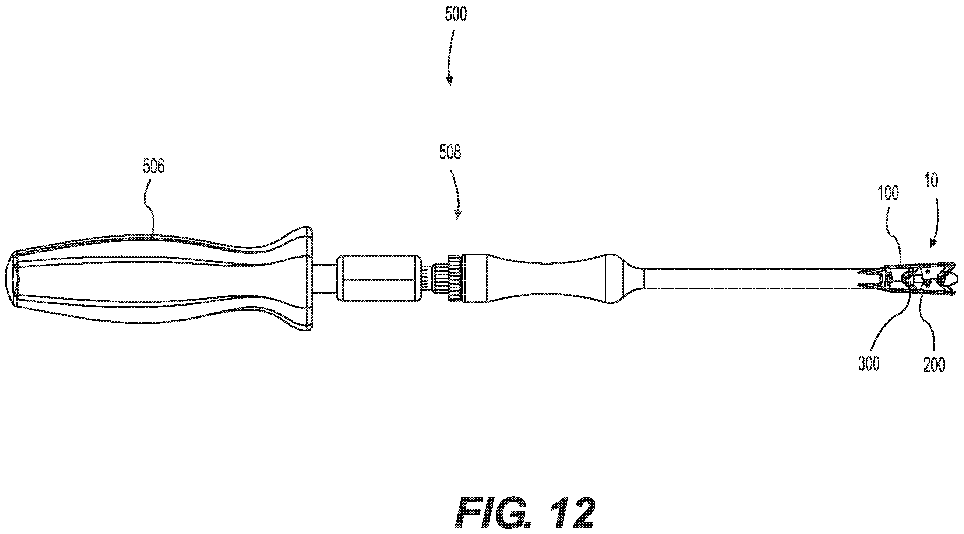

In one aspect, as shown in Figures 8-12, the inter-body fusion device 10 can be actuated by a device driver 500. The device driver can comprise a first member driver 502 sized and shaped to engage the distal end 319 of the first threaded shaft 318, and a second member driver 504 sized and shaped to engage the distal end 337 of the second threaded shaft 336. In another aspect, the first member driver can be a separate tool than the second member driver. Optionally, however, the first member driver 502 and the second member driver 504 can be integrally formed as illustrated in Figure 12. For example, the device driver 500 can further comprise a handle 506 and a clutch collar 508 that allows the device driver to be adjustable between an engaged position and a disengaged position. In the engaged position, the clutch collar can couple the first member driver 502 to the second member driver 504 so that, upon rotation of the handle, both the first member driver and the second member driver rotate at the same speed as the handle. In the disengaged position, the clutch collar 508 can disengage the first member driver 502 from the second member driver 504 so that upon rotation of the handle 506, either the first member driver or the second member driver can rotate at the same speed as the handle while the disengaged driver does not rotate.

In use, the first member driver 502 can be inserted through the longitudinal duct 339 of the second threaded shaft 336 and through the longitudinal pathway 334 of the second member 304 so that the first member driver can be coupled to the distal end 319 of the first threaded shaft 318 of the first member. The second member driver can be coupled to the distal end 337 of the second member. A gripping element 510 of the device driver can grip at least a portion of the inter-body fusion device 10, such as, the first plate 100, the second plate 200, or the insert, such as the second retainer 338 of the insert 300. The clutch collar 508 of the device driver can be placed in the engaged position, and the handle 506 of the device driver can be rotated. For example, if the inter-body fusion device is in the first, unexpanded position, rotation of the handle can cause the first member 302 and the second member 304 of the insert to move from the first insert position towards the second insert position. As the first member and the second member move towards the second insert position, the first plate 100 and the second plate 200 are urged away from each other, and the height of the device increases. In another example, if the inter-body fusion device is in the second expanded position, rotation of the handle can cause the first member 302 and the second member 304 of the insert to move from the second insert position towards the first insert position. As the first member and the second member move towards the first insert position, the first plate 100 and the second plate 200 can move towards each other, and the height of the device decreases.

When the desired device height has been reached, the clutch collar can be moved to the disengaged position. In the disengaged position, rotation of the handle can cause only one of the first member 302 and the second member 304 to move. For example, rotation of the handle in a first direction can cause the first member 302 to move longitudinally towards the trailing edge 104 of the first plate, thereby increasing the angle between the longitudinal axis of the first plate and the second plate (the device angle). In another example, rotation of the first member driver 502 in a second direction that is opposed to the first direction can cause the first member 302 to move longitudinally towards the leading edge 102 of the first plate 100, thereby decreasing the device angle. When the desired device angle has been reached, the device driver 500 can be removed from the device 10.

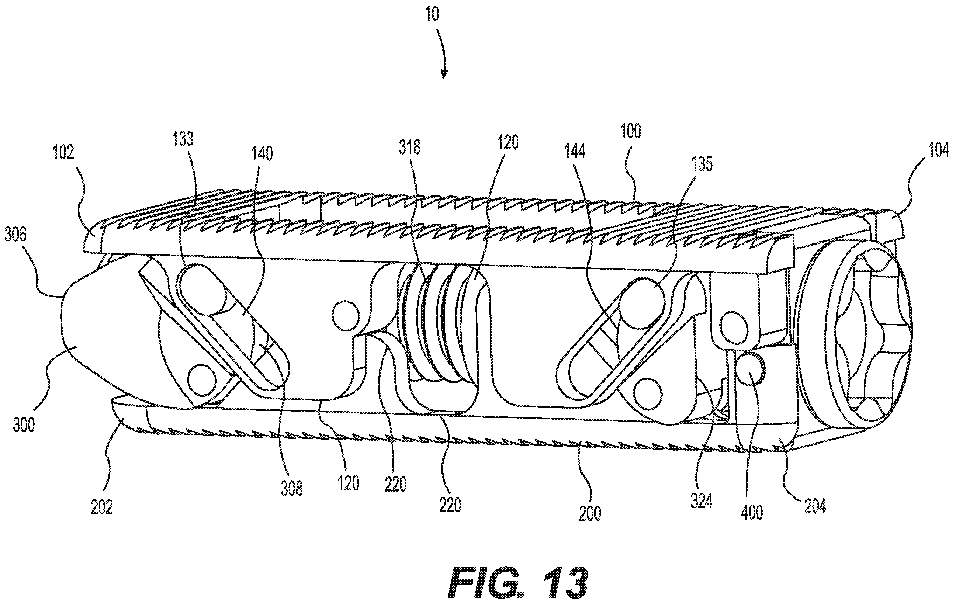

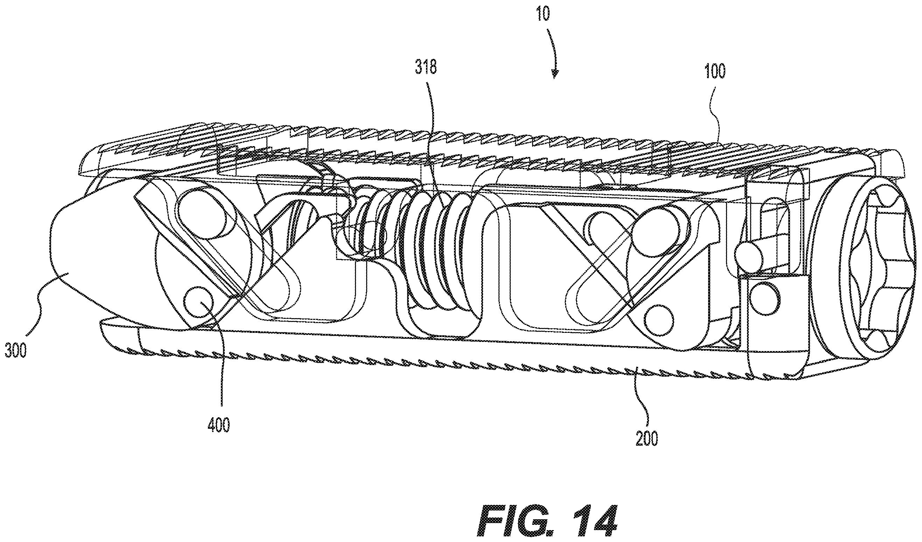

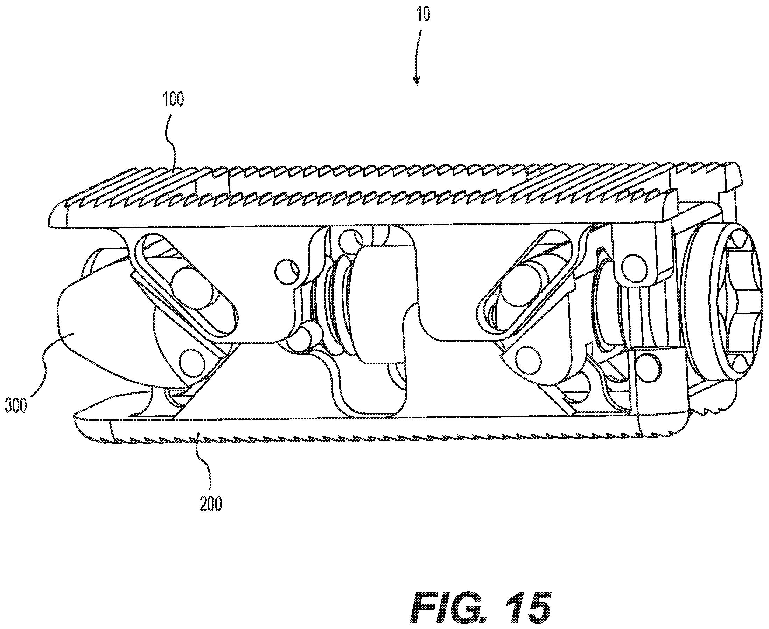

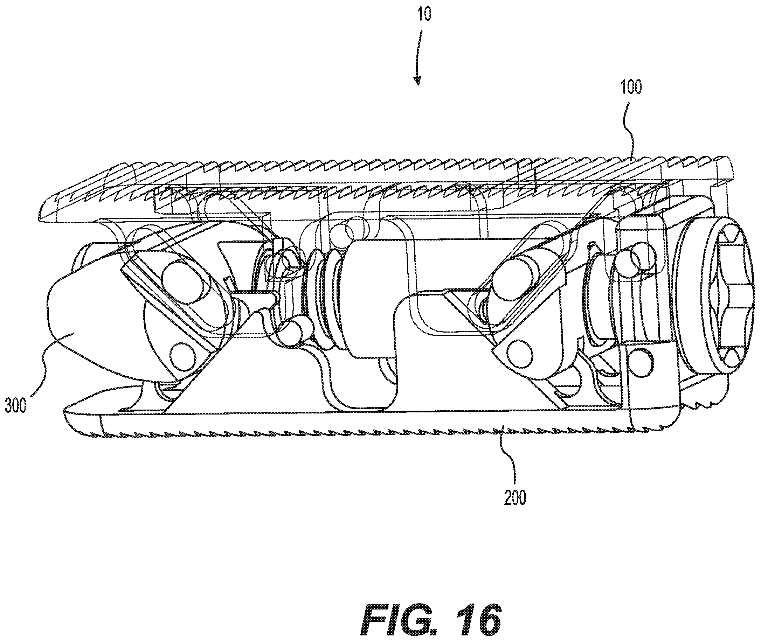

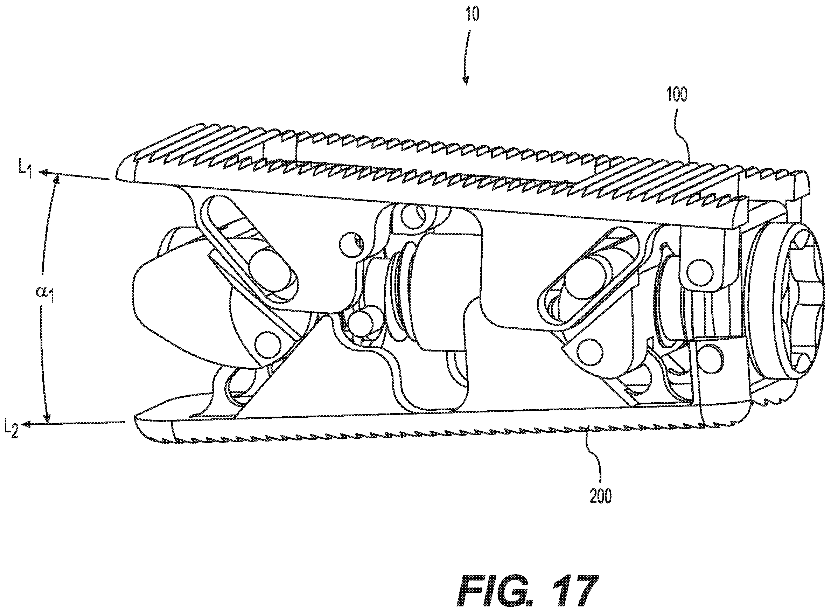

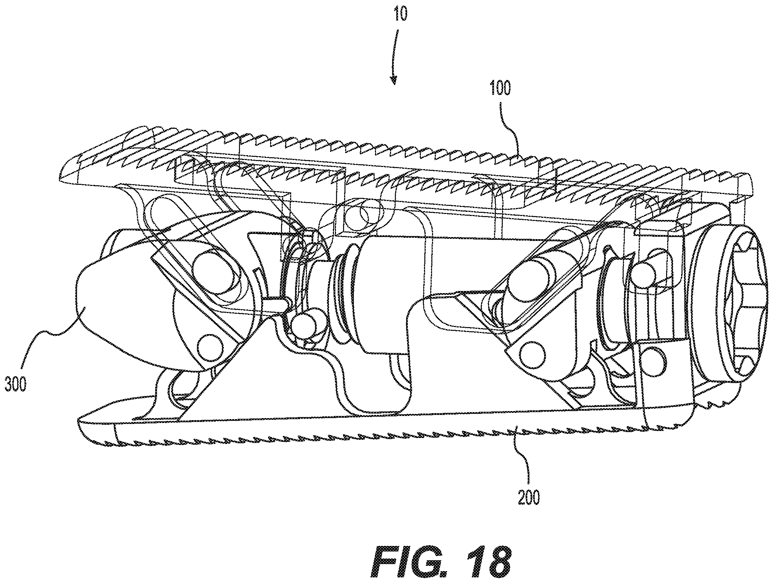

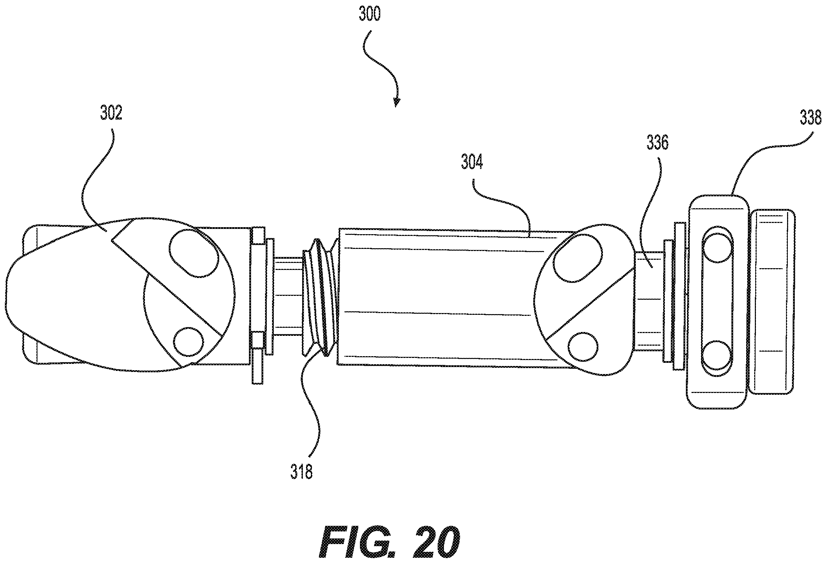

A second embodiment of the inter-body fusion device 10 is illustrated in Figures 13-20, according to one aspect. In this embodiment, the inter-body fusion device can be as described above, comprising a first plate 100, a second plate 200, and an insert 300. Optionally, however, in this embodiment, the insert can be a continuous insert. That is, a portion of the first member 302 of the insert 300 can be coupled to a portion of the second member 304 as illustrated in Figure 19. In one aspect, the first threaded shaft 318 of the first member can be configured to matingly engage a portion of the second bore 332 of the second member such that rotation of the first threaded shaft can move the first member 302 longitudinally relative to the second member 304. For example, at least a portion of the second bore can be threaded so that rotation of the first threaded shaft 318 can cause the distance between the trailing edge 308 of the first member and the leading edge 322 of the second member to change. For example, rotation of the first threaded shaft in a first direction can make the distance between the trailing edge of the first member 302 and the leading edge of the second member 304 smaller. In another example, rotation of the first threaded shaft 318 in a second direction that is opposed to the first direction can make the distance between the trailing edge 308 of the first member and the leading edge of the second member 304 larger.

Referring still to Figure 19, a notch 344 can be defined in a portion of the first member 302, such as, for example, in the upper plate contact surface 310 and/or the lower plate contact surface 312. In one aspect, the notch can be in communication with the first bore 316 so that the first threaded shaft 318 can be inserted through the notch and into the first bore in a direction from the leading edge 306 to the trailing edge 308 of the first member. In another aspect, the first threaded shaft can be inserted through the notch and through the first bore so that at least a portion of the distal end 319 of the first threaded shaft 318 can engage the threads of the second bore 332 of the second member 304.

Assembly of the inter-body fusion device 10 according to this embodiment can be similar to that described above. In order to selectively expand the inter-body fusion device 10 of Figures 13-20 about and between the first unexpanded position and the second expanded position, at least the one of first member 302 or the second member 304 of the insert 300 can be moved longitudinally about and between a first insert position and a second insert position. In one aspect, in the first insert position, the trailing edge 308 of the first member can be spaced from the trailing edge 104 of the first plate 100 an unexpanded first distance, and the trailing edge 324 of the second member can be spaced from the trailing edge 104 of the first plate 100 an unexpanded second distance. In the second insert position, the trailing edge 308 of the first member can be spaced from the trailing edge 104 of the first plate 100 an expanded first distance that is different than the unexpanded first distance, and the trailing edge 324 of the second member can be spaced from the trailing edge 104 of the first plate 100 an expanded second distance that is different than the unexpanded second distance. With regards to the device 10 of Figures 13-20, it can be seen that the expanded first distance is less than the unexpanded first distance, and the expanded second distance is greater than the unexpanded second distance.

In one aspect, in the first unexpanded position, a portion of the longitudinal sidewall 130 of the first plate 100 and the longitudinal sidewall 220 of the second plate 200 can slide by each other until the tab 360 and/or a portion of a pin contacts the leading end 133 of the first slot 140 and/or the trailing end 135 of the second slot 144 and/or, the central portion 406 of a pin positioned in the slot 232 of the second plate contacts the leading end 233 of the first slot 240 and/or the trailing end 235 of the second slot 244. In the second expanded position, a portion of the longitudinal sidewall 130 of the first plate 100 and the longitudinal sidewall 220 of the second plate 200 can slide by each other until the tab 360 and/or a portion of a pin contacts the trailing end 135 of the first slot 140 and/or the leading end 133 of the second slot 144 and/or, the central portion 406 of a pin positioned in the slot 232 of the second plate contacts the trailing end 235 of the first slot 240 and/or the leading end 233 of the second slot 244.

When adjusting the inter-body fusion device 10 of this embodiment about and between the first unexpanded position and the second expanded position, the first member 302 and the second member 304 of the insert 300 do not necessarily need be moved simultaneously or to the same insert position. For example, the first member can be in the first insert position while the second member can be in the second insert position. In another example, the first member 302 can be in the second insert position while the second member 304 can be in the first insert position. Thus, the first member and the second member can be in any insert position between the first insert position and the second insert position at any time regardless of the position of the other member.

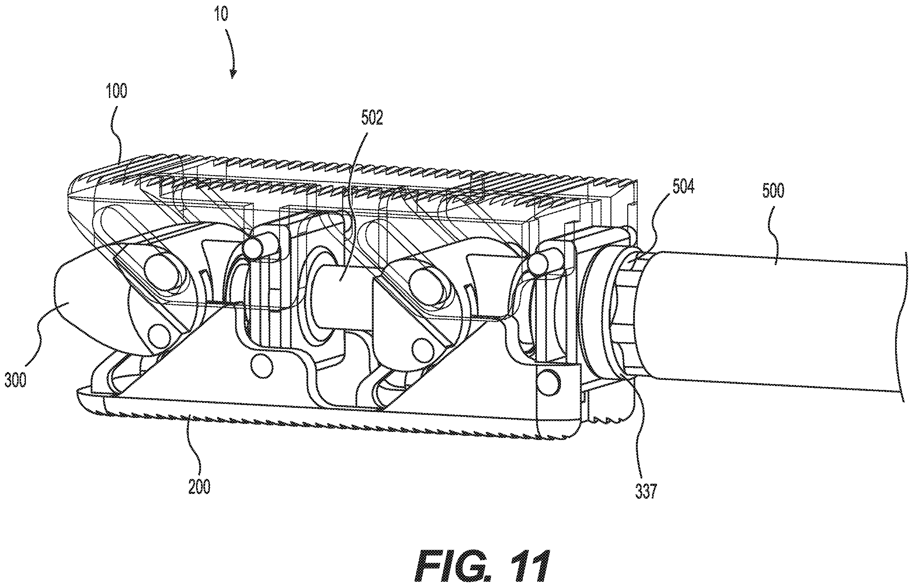

In use, the second member driver 504 can be coupled to the distal end 337 of the second member 304 (as illustrated in Figure 11). If the device is in the first, unexpanded position, rotation of the handle 506 can cause the first member 302 and the second member 304 of the insert to move from the first insert position towards the second insert position, thereby urging the first plate and second plate away from each other. Upon reaching the desired device height, the second member driver can be removed from the second member 304, and the first member driver 502 can be inserted through the longitudinal duct 339 of the second threaded shaft 336 of the second member and through the longitudinal pathway 334 of the second member so that the first member driver can be coupled to the distal end 319 of the first threaded shaft 318 of the first member 302. Rotation of the first member driver can cause the first member to move longitudinally, thereby changing the angle between the first plate and the second plate (the device angle). For example, rotation of the first member driver in a first direction can cause the first member 302 to move longitudinally towards the trailing edge 104 of the first plate, thereby increasing the device angle. In another example, rotation of the first member driver 502 in a second direction that is opposed to the first direction can cause the first member 302 to move longitudinally towards the leading edge 102 of the first plate 100, thereby decreasing the device angle. When the desired device angle has been reached, the first member driver can be removed from the device 10.

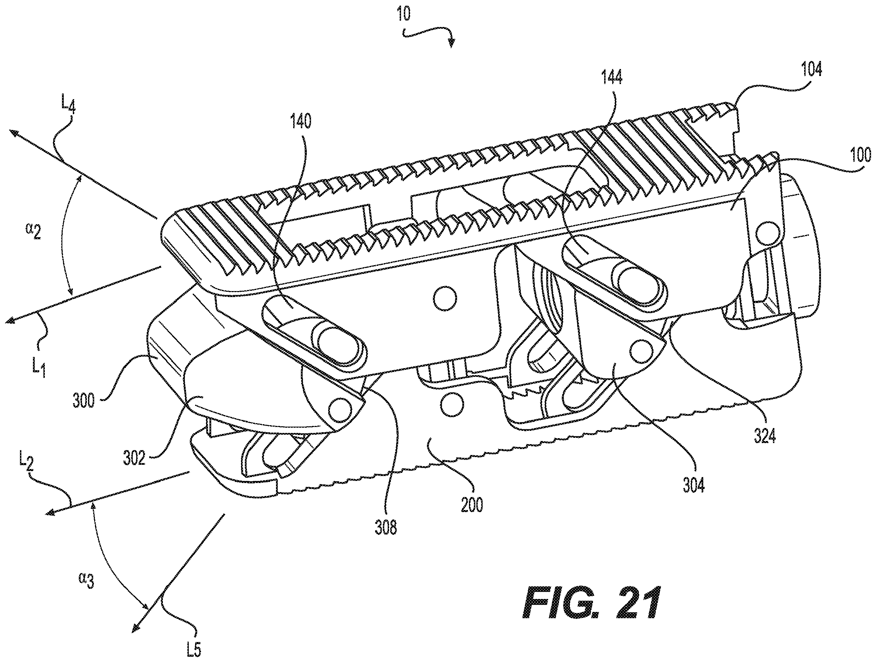

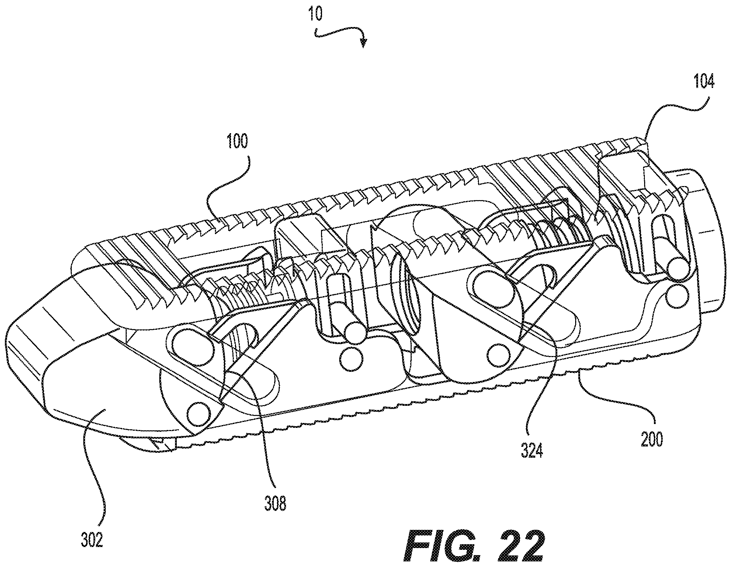

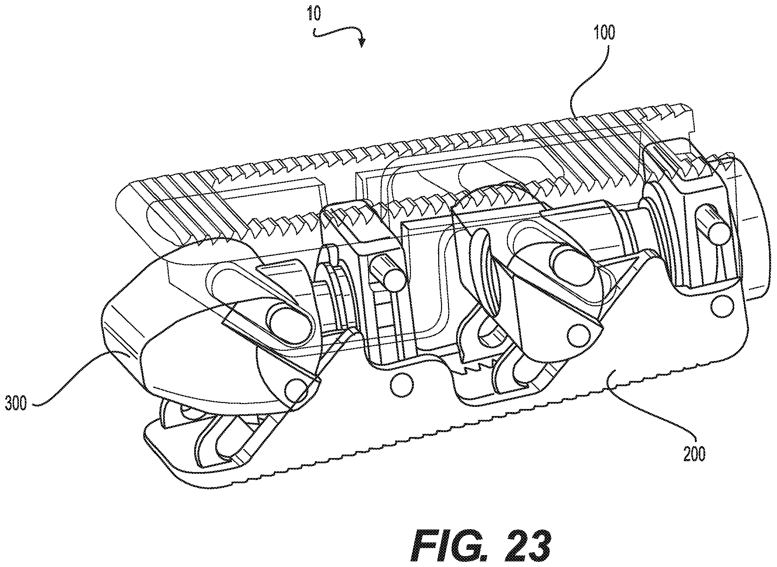

A third embodiment of the inter-body fusion device 10 is illustrated in Figures 21-23. In this embodiment, optionally, the slot axis L₄ of the first inclined slot 140 and the slot axis of the second inclined slot 144 of the first plate can be substantially parallel to each other. That is, the surface angle α₂ between the slot axis L₄ of the first slot and the longitudinal axis L₁ of the first plate 100 can be substantially the same as the surface angle α₂ between the slot axis L₄ of the second slot and the longitudinal axis L₁ of the first plate. In another aspect, the slot axis L₅ of the first inclined slot 240 and the second inclined slot 244 of the second plate can be substantially parallel to each other. That is, the surface angle α₄ between the slot axis L₅ of the first slot and the longitudinal axis L₂ of the second plate 200 can be substantially the same as the surface angle α₄ between the slot axis L₅ of the second slot and the longitudinal axis L₁ of the second plate.

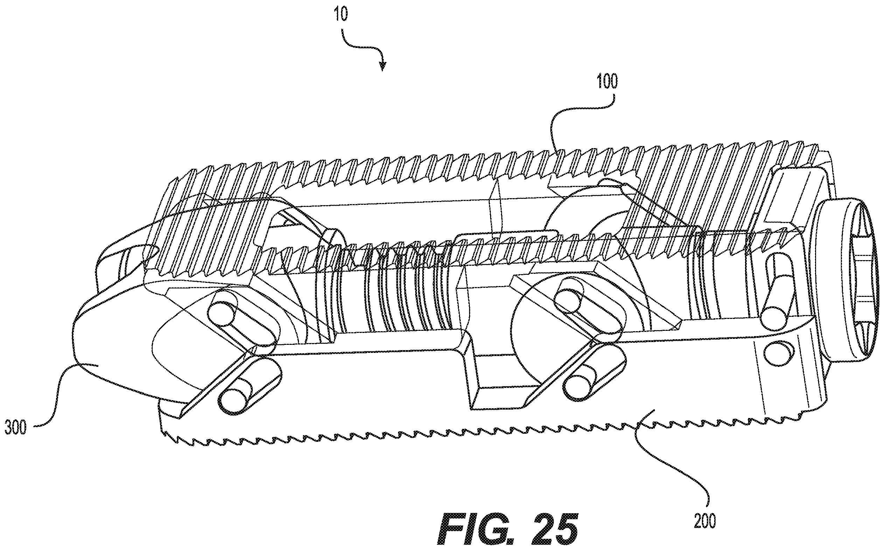

In order to selectively expand the inter-body fusion device 10 of Figures 21-23 about and between the first unexpanded position and the second expanded position, at least one of the first member 302 or the second member 304 of the insert 300 can be moved longitudinally about and between the first insert position and the second insert position. In one aspect, in the first insert position, the trailing edge 308 of the first member can be spaced from the trailing edge 104 of the first plate 100 an unexpanded first distance, and the trailing edge 324 of the second member can be spaced from the trailing edge 104 of the first plate 100 an unexpanded second distance. In the second insert position, the trailing edge 308 of the first member can be spaced from the trailing edge 104 of the first plate 100 an expanded first distance that is different than the unexpanded first distance, and the trailing edge 324 of the second member can be spaced from the trailing edge 104 of the first plate 100 an expanded second distance that is different than the unexpanded second distance. With regards to the device 10 of Figures 21-26, it can be seen that the expanded first distance is less than the unexpanded first distance, and the expanded second distance is less than the unexpanded second distance.

In one aspect, in the first unexpanded position, a portion of the longitudinal sidewall 130 of the first plate 100 and the longitudinal sidewall 220 of the second plate 200 can slide by each other until the tab 360 and/or a portion of a pin contacts the leading end 133 of the first slot 140 and/or the leading end of the second slot 144 and/or, the central portion 406 of a pin positioned in the slot 232 of the second plate contacts the leading end 233 of the first slot 240 and/or the leading end of the second slot 244. In the second expanded position, a portion of the longitudinal sidewall 130 of the first plate 100 and the longitudinal sidewall 220 of the second plate 200 can slide by each other until the tab 360 and/or a portion of a pin contacts the trailing end 135 of the first slot 140 and/or the trailing end of the second slot 144 and/or, the central portion 406 of a pin positioned in the slot 232 of the second plate contacts the trailing end 235 of the first slot 240 and/or the trailing end of the second slot 244.

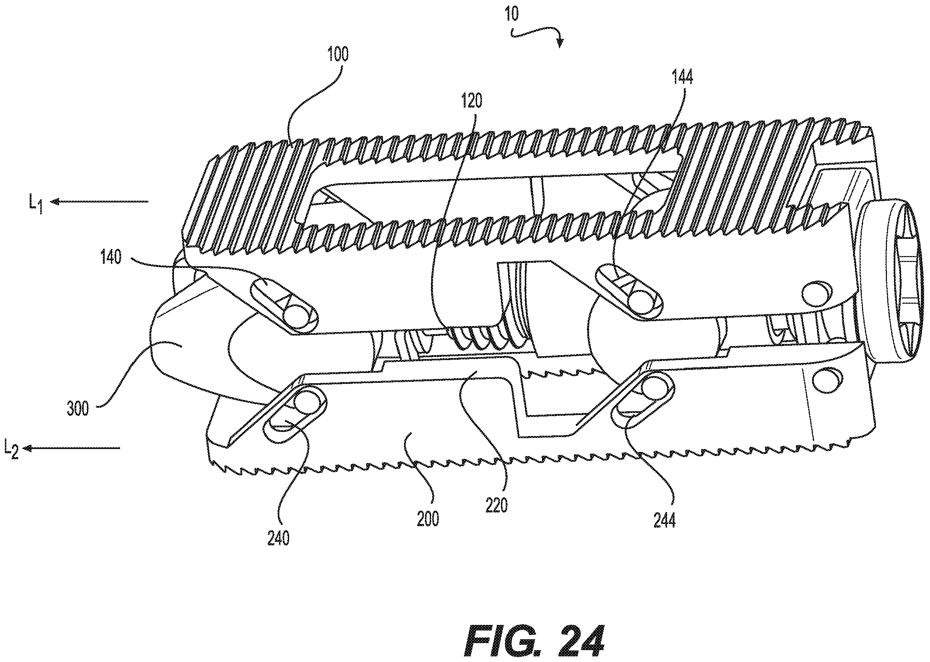

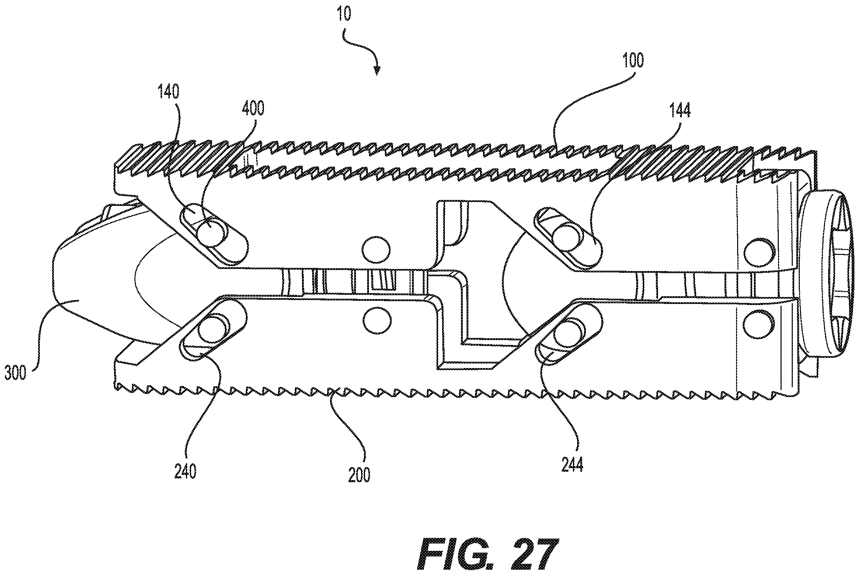

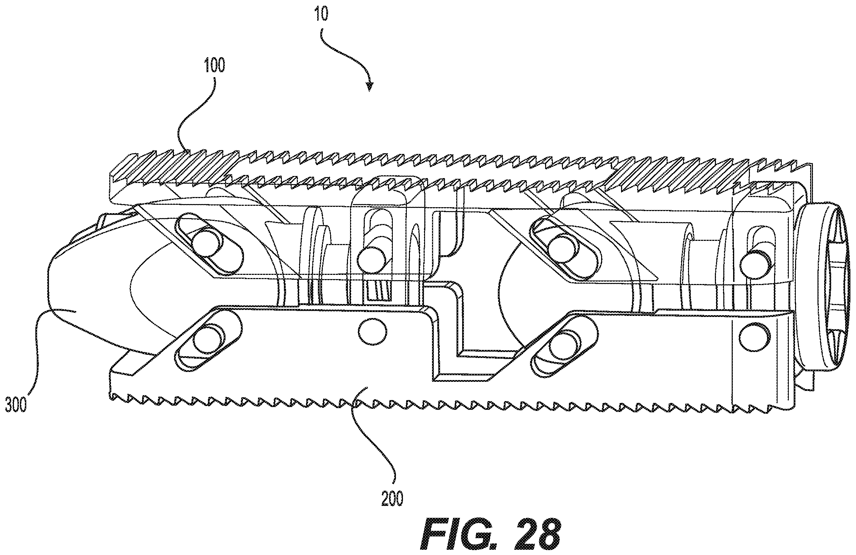

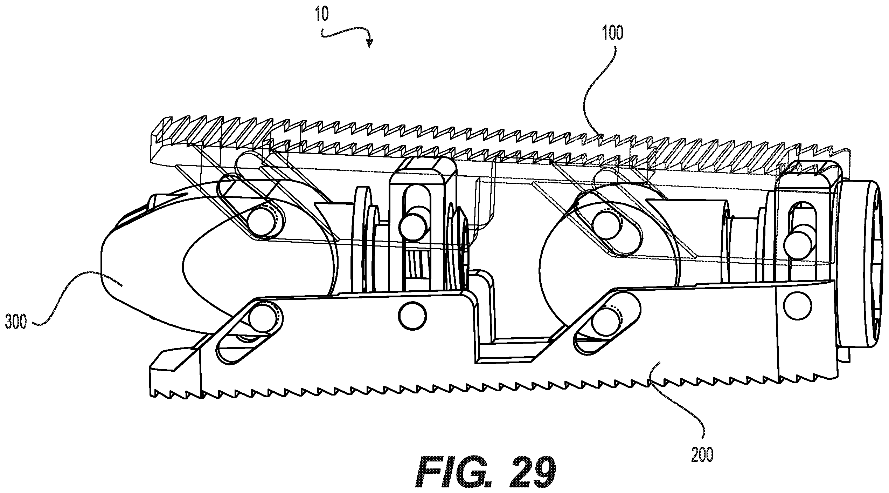

A fourth embodiment of the inter-body fusion device 10 is illustrated in Figures 24-29. In this embodiment, the at least one tab 360 of the insert can be replaced with a pin 400 formed integrally with or coupled to the longitudinal sidewall 330 of the insert 300, according to one aspect. In another aspect, the longitudinal axis of the first inclined slot 140 of the first plate 100 can be substantially parallel to the longitudinal axis of the second inclined slot 144 of the first plate. In a further aspect, the longitudinal axis of the first inclined slot 240 of the second plate 200 can be substantially parallel to the longitudinal axis of the second inclined slot 244 of the second plate. In yet another aspect, the slot length of at least one of the first inclined slots 140, 240 and the second inclined slots 144, 244 can be less than the slot length of the embodiments of the device illustrated in Figures 1-23. That is, the length of the at least one slot 132 of the first plate and/or the at least one slot 232 of the second plate can be varied as desired to limit or increase the range of motion of the device 10. For example, in the device of Figures 24-29, the smaller slot lengths can decrease the overall size of the device in the second expanded position relative to a device having greater slot lengths.

To assemble the inter-body fusion device 10 according to this embodiment, the insert 300 can be positioned between the first plate 100 and the second plate 200 such that the leading edge 306 of the insert, the leading edge 102 of the first plate, and the leading edge 202 of the second plate are facing the same direction. In one aspect, portions of the first plate 100 can overlie the second plate 200. Correspondingly, in one aspect, each longitudinal sidewall 130 of the first plate 100 can substantially align with a longitudinal sidewall 230 of the second plate 200. For example, each longitudinal sidewall of the first plate can substantially overlie at least a portion of a longitudinal sidewall of the second plate. Optionally, each longitudinal sidewall 130 of the first plate 100 can be positioned adjacent to at least a portion of a longitudinal sidewall 230 of the second plate 200 so that at least a portion of the inner surface 120 of the first plate and the inner surface 220 of the second plate can contact each other.

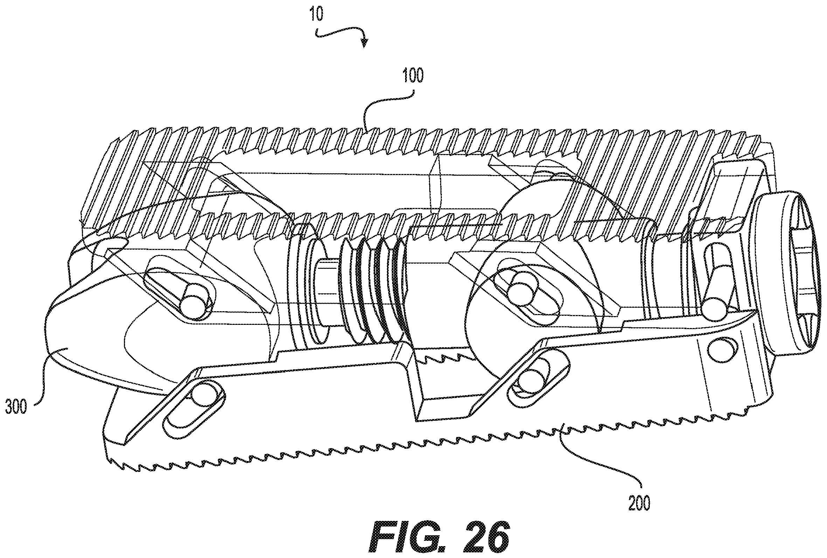

Note that the device according to the embodiment of Figures 24-29 can comprise a portion of the first member 302 of the insert coupled to the second member 304, as illustrated in Figures 24-26, or alternatively, the first member can be physically separate from the second member, as illustrated in Figures 26-29.

Also presented herein are methods of using an inter-body fusion device 10 during an inter-body fusion procedure. In one aspect, the method comprises accessing the desired disc space, choosing the inter-body fusion device size with the appropriate height, inserting the inter-body fusion device 10 into the desired area in the disc space, expanding the inter-body fusion device from the first unexpanded position to the second expanded position with longitudinal movement of the insert 300, and adjusting the angle of the of the first plate 100 relative to the second plate. An additional step of packing the interior cavity 15 via the longitudinal duct 339 of the second threaded shaft 336 and the longitudinal pathway 334 of the second member 304 with bone fusion material after expansion is also contemplated. In one aspect, the method of using an inter-body fusion device 10 during an inter-body fusion procedure further comprises the step of securing the insert to the first and second plates. In another aspect, the method of using an inter-body fusion device during an inter-body fusion procedure further comprises the step of securing the inter-body fusion device 10 to the surrounding bony structure.

In one aspect, the step of choosing the inter-body fusion device 10 size with the appropriate height and angle comprises placing an undersized trial device in the disc space, expanding the trial device to the second expanded position, and repeating until the correct height and lordosis is found. The trial height and angle gives the information to prescribe the correct inter-body fusion device for the procedure. In another aspect, the method further comprises selecting a desired embodiment of inter-body fusion device, as described above.

Although several aspects of the invention have been disclosed in the foregoing specification, it is understood by those skilled in the art that many modifications and other aspects of the invention will come to mind to which the invention pertains, having the benefit of the teaching presented in the foregoing description and associated drawings. It is thus understood that the invention is not limited to the specific aspects disclosed hereinabove, and that many modifications and other aspects are intended to be included within the scope of the appended claims. Moreover, although specific terms are employed herein, as well as in the claims that follow, they are used only in a generic and descriptive sense, and not for the purposes of limiting the described invention.

## Claims

1. An inter-body fusion device for use in surgery comprising:
a first plate (100) having a leading edge (102), a trailing edge (104), an upper bone contact surface (110), an opposed first plate inner surface (120), and a first plate longitudinal axis (L1);
a second plate (200) having a leading edge (202), a trailing edge (204), a lower bone contact surface (210), an opposed second plate inner surface (210), and a second plate longitudinal axis (L2), wherein the first plate (100) substantially overlies the second plate (200) and is positioned such that the first plate longitudinal axis (L1) and the second plate longitudinal axis (L2) form a device angle; and
an insert (300) comprising a first member (302) having a leading edge (306) and a trailing edge (308), and a second member (304) having a leading edge (322) and a trailing edge (324), the insert (300) positioned substantially therebetween the first plate (100) and the second plate (200),
wherein movement of the first member(302) longitudinally with respect to the first and second plates (100, 200) increases a distance between a portion of the leading edge (102, 202) of the first and second plates (100, 200) and movement of the second member (304) longitudinally with respect to the first and second plates (100, 200) increases the distance between a portion of the trailing edge (104, 204) of the first and second plates (100, 200),
**characterized in that**
both the first member (302) and the second member (304) each include an upper plate contact surface (310, 326) and a lower plate contact surface (312, 328), and
the first and second members (302, 304) are physically separate and operate independently and are configured to allow selectively altering both the distance between the first plate (100) and the second plate (200) and the device angle.

2. The inter-body fusion device of claim 1, wherein the device angle is substantially 0 degrees such that the first plate (100) and the second plate (200) are substantially parallel to each other and the first member (302) comprises a first retainer (320) configured to couple a portion of the first member (302) to at least one of the first plate (100) and the second plate (200).

3. The inter-body fusion device of claim 1, wherein the device angle is an acute angle between about 1 degree and about 45 degrees.

4. The inter-body fusion device of claim 1, wherein the device angle is an acute angle between about 5 degrees and about 30 degrees.

5. The inter-body fusion device of claim 1, wherein the first plate (100) comprises a pair of longitudinal sidewalls (130) extending from a portion of the first plate inner surface (120), and the second plate (200) comprises a pair of longitudinal sidewalls (230) extending from a portion of the second plate inner surface (220), and wherein one of the pairs of longitudinal sidewalls (130, 230) is positioned within the other pair of longitudinal sidewalls.

6. The inter-body fusion device of claim 5, wherein the longitudinal sidewall (130) of the first plate (100) defines a first inclined slot (140) and a second inclined slot (144), each slot having a leading end (133) and a trailing end (135), the leading end (133) being positioned closer to the leading edge (102) of the first plate (100).

7. The inter-body fusion device of claim 6, wherein the first slot (140) is defined along a first slot axis (L4) which is positioned at an acute angle relative to the longitudinal axis (L1) of the first plate (100), and wherein the second slot (144) is defined along a second slot axis which is positioned at an acute angle relative to the longitudinal axis (L1) of the first plate (100).

8. The inter-body fusion device of claim 6, wherein the longitudinal sidewall (230) of the second plate (200) defines a third inclined slot (240) and a fourth inclined slot (244), each slot having a leading end (233) and a trailing end (235).

9. The inter-body fusion device of claim 8, wherein the third slot (240) is defined along a third slot axis (L5) which is positioned at an acute angle relative to the longitudinal axis (L2) of the second plate (200), and wherein the fourth slot (244) is defined along a fourth slot axis which is positioned at an acute angle relative to the longitudinal axis (L2) of the second plate (200).

10. The inter-body fusion device of claim 1, wherein portions of the first member (302) are positioned and configured to act on portions of the leading edge (102) of the first plate (100) and portions of the leading edge (202) of the second plate (200) to facilitate expanding portions of the inter-body fusion device by selectively separating portions of the leading edges (102, 202) of the first and second plates (100, 200).

11. The inter-body fusion device of claim 10, wherein portions of the second member (304) are positioned and configured to act on portions of the trailing edge (104) of the first plate (100) and portions of the trailing edge (204) of the second plate (200) to facilitate expanding portions of the inter-body fusion device by selectively separating portions of the trailing edges (104, 204) of the first and second plates (100, 200).

12. The inter-body fusion device of claim 1, wherein the trailing edge (104) of the first member (302) defines a first bore (316) configured to engage a threaded shaft (318), wherein rotation of the threaded shaft (318) in a first direction moves the first member (302) proximally and rotation of the threaded shaft (318) in a second direction moves the first member (302) distally.

13. The inter-body fusion device of claim 12, wherein the second member (304) defines a second bore (332) that extends longitudinally through the second member (304), the second bore (332) configured to engage a second threaded shaft (336), wherein rotation of the second threaded shaft (336) in a first direction moves the second member (304) proximally and rotation of the second threaded shaft (336) in a second direction moves the second member (304) distally.

14. The inter-body fusion device of claim 13, wherein a distal end (337) of the second threaded shaft (336) defines a feature to engage an actuation device, such that rotation of the actuation device can rotate the second threaded shaft (336).

15. The inter-body fusion device of claim 14, wherein a longitudinal duct (339) is defined therethrough the second threaded shaft (336) configured to enable at least a portion of the actuation device to be inserted through the longitudinal duct (339).

## Patentansprüche

1. Intersomatische Fusionsvorrichtung zur Verwendung in der Chirurgie, welches Folgendes aufweist:
eine erste Platte (100), die einen vorderen Rand (102), einen hinteren Rand (104), eine obere Knochenkontaktfläche (110), eine gegenüberliegende erste innere Plattenfläche (120) und eine erste Plattenlängsachse (L1) aufweist;
eine zweite Platte (200), die einen vorderen Rand (202), einen hinteren Rand (204), eine untere Knochenkontaktfläche (210), eine gegenüberliegende zweite innere Plattenfläche (210) und eine zweite Plattenlängsachse (L2) aufweist,
wobei die erste Platte (100) im Wesentlichen über der zweiten Platte (200) angeordnet und derart positioniert ist, dass die erste Plattenlängsachse (L1) und die zweite Plattenlängsachse (L2) einen Vorrichtungswinkel bilden; und
einen Einsatz (300), der ein erstes Element (302) mit einem vorderen Rand (306) und einem hinteren Rand (308) und ein zweites Element (304) mit einem vorderen Rand (322) und einem hinteren Rand (324) aufweist, wobei der Einsatz (300) im Wesentlichen zwischen der ersten Platte (100) und der zweiten Platte (200) positioniert ist,
wobei eine Bewegung des ersten Elements (302) in Längsrichtung in Bezug auf die ersten und zweiten Platten (100,200) einen Abstand zwischen einem Abschnitt des vorderen Rands (102,202) der ersten und zweiten Platten (100,200) vergrößert und eine Bewegung des zweiten Elements (304) in Längsrichtung in Bezug auf die ersten und zweiten Platten (100,200) den Abstand zwischen einem Abschnitt des hinteren Rands (104,204) der ersten und zweiten Platten (100,200) vergrößert,
**dadurch gekennzeichnet, dass**
sowohl das erste Element (302) als auch das zweite Element (304) jeweils eine obere Plattenkontaktfläche (310,326) und eine untere Plattenkontaktfläche (312,328) aufweisen, und
die ersten und zweiten Elemente (302,304) körperlich getrennt sind und unabhängig voneinander betrieben werden und dafür vorgesehen sind, es wahlweise zu erlauben, sowohl den Abstand zwischen der ersten Platte (100) und der zweiten Platte (200) als auch den Vorrichtungswinkel zu ändern.

2. Intersomatische Fusionsvorrichtung nach Anspruch 1, wobei der Vorrichtungswinkel im Wesentlichen 0 Grad ist, so dass die erste Platte (100) und die zweite Platte (200) im Wesentlichen parallel zueinander sind, und wobei das erste Element (302) ein erstes Halteelement (320) aufweist, das dafür vorgesehen ist, einen Abschnitt des ersten Elements (302) mit wenigstens einer der ersten Platte (100) und der zweiten Platte (200) zu verbinden.

3. Intersomatische Fusionsvorrichtung nach Anspruch 1, wobei der Vorrichtungswinkel ein spitzer Winkel zwischen ungefähr 1 Grad und ungefähr 45 Grad ist.

4. Intersomatische Fusionsvorrichtung nach Anspruch 1, wobei der Vorrichtungswinkel ein spitzer Winkel zwischen ungefähr 5 Grad und ungefähr 30 Grad ist.

5. Intersomatische Fusionsvorrichtung nach Anspruch 1, wobei die erste Platte (100) ein Paar länglicher Seitenwände (130) aufweist, die sich von einem Abschnitt der ersten inneren Plattenfläche (120) erstrecken, und wobei die zweite Platte (200) ein Paar länglicher Seitenwände (230) aufweist, die sich von einem Abschnitt der zweiten inneren Plattenfläche (220) erstrecken, und wobei eines der Paare von länglichen Seitenwänden (130,230) innerhalb des anderen Paares von länglichen Seitenwänden positioniert ist.

6. Intersomatische Fusionsvorrichtung nach Anspruch 5, wobei die längliche Seitenwand (130) der ersten Platte (100) einen ersten geneigten Schlitz (140) und einen zweiten geneigten Schlitz (144) festlegt, wobei jeder Schlitz einen vorderen Rand (133) und einen hinteren Rand (135) aufweist, wobei der hintere Rand (133) näher an dem vorderen Rand (102) der ersten Platte (100) positioniert ist.

7. Intersomatische Fusionsvorrichtung nach Anspruch 6, wobei der erste Schlitz (140) entlang einer ersten Schlitzachse (L4) festgelegt ist, die in einem spitzen Winkel relativ zu der Längsachse (L1) der ersten Platte (100) positioniert ist, und wobei der zweite Schlitz (144) entlang einer zweiten Schlitzachse festgelegt ist, die in einem spitzen Winkel relativ zu der Längsachse (L1) der ersten Platte (100) positioniert ist.

8. Intersomatische Fusionsvorrichtung nach Anspruch 6, wobei die längliche Seitenwand (230) der zweiten Platte (200) einen dritten geneigten Schlitz (240) und einen vierten geneigten Schlitz (244) festlegt, wobei jeder Schlitz ein vorderes Ende (233) und ein hinteres Ende (235) aufweist.

9. Intersomatische Fusionsvorrichtung nach Anspruch 8, wobei der dritte Schlitz (240) entlang einer dritten Schlitzachse (L5) festgelegt ist, die in einem spitzen Winkel relativ zu der Längsachse (L2) der zweiten Platte (200) positioniert ist, und wobei der vierte Schlitz (244) entlang einer vierten Schlitzachse festgelegt ist, die in einem spitzen Winkel relativ zu der Längsachse (L2) der zweiten Platte (200) positioniert ist.

10. Intersomatische Fusionsvorrichtung nach Anspruch 1, wobei Abschnitte des ersten Elements (302) so positioniert und dafür ausgebildet sind, auf Abschnitte des vorderen Rands (102) der ersten Platte (100) und auf Abschnitte des vorderen Rands (202) der zweiten Platte (200) zu wirken, um das Verlängern von Abschnitten der intersomatischen Fusionsvorrichtung durch wahlweises Trennen von Abschnitten der vorderen Ränder (102,202) der ersten und zweiten Platten (100,200) zu erleichtern.

11. Intersomatische Fusionsvorrichtung nach Anspruch 10, wobei Abschnitte des zweiten Elements (304) so positioniert und dafür ausgebildet sind, auf Abschnitte des hinteren Rands (104) der ersten Platte (100) und auf Abschnitte des hinteren Rands (204) der zweiten Platte (200) zu wirken, um das Verlängern von Abschnitten der intersomatischen Fusionsvorrichtung durch wahlweises Trennen von Abschnitten der hinteren Ränder (104,204) der ersten und zweiten Platten (100,200) zu erleichtern.

12. Intersomatische Fusionsvorrichtung nach Anspruch 1, wobei der hintere Rand (104) des ersten Elements (302) eine erste Bohrung (316) festlegt, die dafür vorgesehen ist, mit einer mit einem Gewinde versehene Welle (318) in Eingriff zu gehen, wobei eine Rotation der mit einem Gewinde versehenen Welle (318) in einer ersten Richtung das erste Element (302) proximal bewegt und eine Rotation der mit einem Gewinde versehenen Welle (318) in einer zweiten Richtung das erste Element (302) distal bewegt.

13. Intersomatische Fusionsvorrichtung nach Anspruch 12, wobei das zweite Element (304) eine zweite Bohrung (332) festlegt, die sich in Längsrichtung durch das zweite Element (304) erstreckt, wobei die zweite Bohrung (332) dafür vorgesehen ist, mit einer zweiten mit einem Gewinde versehenen Welle (336) in Eingriff zu gehen, wobei eine Rotation der zweiten mit einem Gewinde versehenen Welle (336) in einer ersten Richtung das zweite Element (304) proximal bewegt und eine Rotation der zweiten mit einem Gewinde versehenen Welle (336) in einer zweiten Richtung das zweite Element (304) distal bewegt.

14. Intersomatische Fusionsvorrichtung nach Anspruch 13, wobei ein distales Ende (337) der zweiten mit einem Gewinde versehenen Welle (336) ein Merkmal aufweist, um mit einer Betätigungseinrichtung in Eingriff zu gehen, so dass eine Rotation der Betätigungseinrichtung die zweite mit einem Gewinde versehene Welle (336) rotieren kann.

15. Intersomatische Fusionsvorrichtung nach Anspruch 14, wobei durch die zweite mit einem Gewinde versehenen Welle (336) eine längliche Leitung (339) festgelegt ist, die dafür vorgesehen ist, es wenigstens einem Abschnitt der Betätigungseinrichtung zu erlauben, durch die längliche Leitung (339) eingeführt zu werden.

## Revendications

1. Dispositif d'arthrodèse intersomatique pour utilisation en chirurgie comprenant :
une première plaque (100) ayant un bord avant (102), un bord arrière (104), une surface de contact avec l'os supérieure (110), une première surface interne de plaque opposée (120), et un premier axe longitudinal de plaque (L1) ;
une deuxième plaque (200) ayant un bord avant (202), un bord arrière (204), une surface de contact avec l'os inférieure (210), une deuxième surface interne de plaque opposée (210), et un deuxième axe longitudinal de plaque (L2), où la première plaque (100) recouvre essentiellement la deuxième plaque (200) et est positionnée de sorte que le premier axe longitudinal de plaque (L1) et le deuxième axe longitudinal de plaque (L2) forment un angle de dispositif ; et
un insert (300) comprenant un premier organe (302) ayant un bord avant (306) et un bord arrière (308), et un deuxième organe (304) ayant un bord avant (322) et un bord arrière (324), l'insert (300) étant positionné essentiellement entre la première plaque (100) et la deuxième plaque (200),
dans lequel le déplacement du premier organe (302) longitudinalement par rapport aux première et deuxième plaques (100, 200) augmente la distance entre une partie du bord avant (102, 202) des première et deuxième plaques (100, 200) et le déplacement du deuxième organe (304) longitudinalement par rapport aux première et deuxième plaques (100, 200) augmente la distance entre une partie du bord arrière (104, 204) des première et deuxième plaques (100, 200),
**caractérisé en ce que**
à la fois le premier organe (302) et le deuxième organe (304) comportent chacun une surface de contact de plaque supérieure (310, 326) et une surface de contact de plaque inférieure (312, 328), et
les premier et deuxième organes (302, 304) sont physiquement séparés et fonctionnent indépendamment et sont configurés pour permettre de modifier sélectivement à la fois la distance entre la première plaque (100) et la deuxième plaque (200) et l'angle de dispositif.

2. Dispositif d'arthrodèse intersomatique de la revendication 1, dans lequel l'angle de dispositif est essentiellement égal à 0 degrés de sorte que la première plaque (100) et la deuxième plaque (200) soient essentiellement parallèles l'une à l'autre et le premier organe (302) comprend un premier organe de retenue (320) configuré pour coupler une partie du premier organe (302) à au moins l'une de la première plaque (100) et de la deuxième plaque (200).

3. Dispositif d'arthrodèse intersomatique de la revendication 1, dans lequel l'angle de dispositif est un angle aigu compris entre environ 1 degré et environ 45 degrés.

4. Dispositif d'arthrodèse intersomatique de la revendication 1, dans lequel l'angle de dispositif est un angle aigu compris entre environ 5 degrés et environ 30 degrés.

5. Dispositif d'arthrodèse intersomatique de la revendication 1, dans lequel la première plaque (100) comprend une paire de parois latérales longitudinales (130) s'étendant à partir d'une partie de la première surface interne de plaque (120), et la deuxième plaque (200) comprend une paire de parois latérales longitudinales (230) s'étendant à partir d'une partie de la deuxième surface interne de plaque (220), et dans lequel l'une des paires de parois latérales longitudinales (130, 230) est positionnée à l'intérieur de l'autre paire de parois latérales longitudinales.

6. Dispositif d'arthrodèse intersomatique de la revendication 5, dans lequel la paroi latérale longitudinale (130) de la première plaque (100) définit une première fente inclinée (140) et une deuxième fente inclinée (144), chaque fente ayant une extrémité avant (133) et une extrémité arrière (135), l'extrémité avant (133) étant positionnée plus près du bord avant (102) de la première plaque (100).

7. Dispositif d'arthrodèse intersomatique de la revendication 6, dans lequel la première fente (140) est définie le long d'un premier axe de fente (L4) qui est positionné selon un angle aigu par rapport à l'axe longitudinal (L1) de la première plaque (100), et dans lequel la deuxième fente (144) est définie le long d'un deuxième axe de fente qui est positionné selon un angle aigu par rapport à l'axe longitudinal (L1) de la première plaque (100).

8. Dispositif d'arthrodèse intersomatique de la revendication 6, dans lequel la paroi latérale longitudinale (230) de la deuxième plaque (200) définit une troisième fente inclinée (240) et une quatrième fente inclinée (244), chaque fente ayant une extrémité avant (233) et une extrémité arrière (235).

9. Dispositif d'arthrodèse intersomatique de la revendication 8, dans lequel la troisième fente (240) est définie le long d'un troisième axe de fente (L5) qui est positionné selon un angle aigu par rapport à l'axe longitudinal (L2) de la deuxième plaque (200), et dans lequel la quatrième fente (244) est définie le long d'un quatrième axe de fente qui est positionné selon un angle aigu par rapport à l'axe longitudinal (L2) de la deuxième plaque (200).

10. Dispositif d'arthrodèse intersomatique de la revendication 1, dans lequel des parties du premier organe (302) sont positionnées et configurées pour agir sur des parties du bord avant (102) de la première plaque (100) et des parties du bord avant (202) de la deuxième plaque (200) pour faciliter l'expansion de parties du dispositif d'arthrodèse intersomatique par séparation sélective de parties des bords avant (102, 202) des première et deuxième plaques (100, 200).

11. Dispositif d'arthrodèse intersomatique de la revendication 10, dans lequel des parties du deuxième organe (304) sont positionnées et configurées pour agir sur des parties du bord arrière (104) de la première plaque (100) et des parties du bord arrière (204) de la deuxième plaque (200) pour faciliter l'expansion de parties du dispositif d'arthrodèse intersomatique par séparation sélective de parties des bords arrière (104, 204) des première et deuxième plaques (100, 200).

12. Dispositif d'arthrodèse intersomatique de la revendication 1, dans lequel le bord arrière (104) du premier organe (302) définit un premier alésage (316) configuré pour s'engager avec un arbre fileté (318), dans lequel la rotation de l'arbre fileté (318) dans une première direction déplace le premier organe (302) de manière proximale et la rotation de l'arbre fileté (318) dans une deuxième direction déplace le premier organe (302) de manière distale.

13. Dispositif d'arthrodèse intersomatique de la revendication 12, dans lequel le deuxième organe (304) définit un deuxième alésage (332) qui s'étend longitudinalement à travers le deuxième organe (304), le deuxième alésage (332) étant configuré pour s'engager avec un deuxième arbre fileté (336), dans lequel la rotation du deuxième arbre fileté (336) dans une première direction déplace le deuxième organe (304) de manière proximale et la rotation du deuxième arbre fileté (336) dans une deuxième direction déplace le deuxième organe (304) de manière distale.

14. Dispositif d'arthrodèse intersomatique de la revendication 13, dans lequel une extrémité distale (337) du deuxième arbre fileté (336) définit une caractéristique pour s'engager avec un dispositif d'actionnement, de sorte que la rotation du dispositif d'actionnement puisse faire tourner le deuxième arbre fileté (336).

15. Dispositif d'arthrodèse intersomatique de la revendication 14, dans lequel un conduit longitudinal (339) est défini à travers le deuxième arbre fileté (336) configuré pour permettre à au moins une partie du dispositif d'actionnement d'être insérée à travers le conduit longitudinal (339).
